# EUROPÄISCHE PATENTSCHRIFT

(11) **EP 0 826 692 B1**
(45) Veröffentlichungstag und Bekanntmachung des Hinweises auf die Patenterteilung: **05.03.2003**
(21) Anmeldenummer: 97810582.3
(22) Anmeldetag: 19.08.1997
(51) Int. Cl.: C07F 9/53, C07C 49/82, C07C 49/83, G03F 7/031

(54) **Molekülkomplexverbindungen als Photoinitiatoren**
Molecular complexes as photoinitiators
Complexes moléculaires comme photoinitiateurs

(30) Priorität: 28.08.1996 CH 211596
(43) Veröffentlichungstag der Anmeldung: 04.03.1998
(73) Patentinhaber: Ciba Specialty Chemicals Holding Inc., 4057 Basel (CH)
(72) Erfinder: Leppard, David George, 1723 Marly (CH); James, Thomas Lloyd, 4402 Frenkendorf (CH); Höck, Nils, 79379 Müllheim-Zunzingen (CH); Köhler, Manfred, 79108 Freiburg (DE); Salathé, Ronald, 4312 Magden (DE)

(56) Entgegenhaltungen:
- EP-A- 0 057 474
- EP-A- 0 446 175
- EP-A- 0 670 323
- WO-A-96/07662

## Beschreibung

Die Erfindung betrifft Molekülkomplexverbindungen aus Mono-, Bis- und Trisacylphosphinoxiden mit α-Hydroxyketonen sowie die Verwendung dieser Molekülkomplexverbindungen als Photoinitiatoren.

Acylphosphinoxide sind in der Literatur als Photoinitiatoren beschrieben. So sind zum Beispiet der EP-A 7 508 und der EP-A 057 474 Herstellung und Verwendung einiger Monoacylphosphinoxide zu entnehmen. Weitere Monoacylphosphinoxide sowie Bisacylphosphinoxide sind aus dem US Patent 5,218,009 bekannt. Die Herstellung und Verwendung von Bisacylphosphinoxid-Photoinitiatoren ist z.B. in den US Patenten 4,737,593; 4,792,632; und in der GB-A 2 259 704 offenbart. Trisacylphosphinoxid-Verbindungen sind beispielsweise der WO-A 96/7662 zu entnehmen. α-Hydroxyketon-Verbindungen als Photoinitiatoren sind beispielsweise in den US Patenten 4,347,111 und 4,672,079 sowie in der EP-A 3002 aufgeführt. Die Verwendung von Photoinitiatorgemischen aus Acylphosphinoxiden und α-Hydroxyketonen ist beispielsweise in der GB-A 2 259 704 oder der GB-A 2 292 740 beschrieben. In der EP-A 670 323 werden dimere Bisacylphosphinoxide offenbart, welche gegebenenfalls auch in Kombination mit α-Hydroxyketonen verwendet werden. Die EP-A 446 175 beschreibt Photoinitiatorgemische, welche Mono- oder Bisacylphosphinoxide in Kombination mit einem α-Hydroxyketon und Benzophenon enthalten.

Es besteht ein Bedarf an gut zugänglichen, reaktiven, lagerstabilen und gut in zu polymerisierende Formulierungen einarbeitbaren Photoinitiatorverbindungen.

Es wurde nun gefunden, dass Molekülkomplexverbindungen aus verschiedenen Photoinitiatorverbindungen diese Eigenschaften besitzen.

Gegenstand der Erfindung sind daher Molekülkomplexverbindungen aus einer Mono-, Bisoder Trisacylphosphinoxid-Verbindung mit einer α-Hydroxyketon-Verbindung.

Die Molekülkomplex-Verbindungen lassen sich beispielsweise durch allgemein bekannte Verfahren zur Züchtung von Kristallen herstellen, wie z.B. das Züchten aus Lösung oder Schmelzverfahren. Solche Verfahren der Kristallisation sind dem Fachmann bekannt und auch in Lehrbüchern der Chemie beschrieben, wie beispielsweise in Ullmann's Encyclopedia of Industrial Chemistry, Vol. A 8, 127-131, Verlag Chemie, Weinheim-New York (1987) oder Kirk-Othmer, Encyclopedia of Chemical Technology, Vol. 7, 672-81, Verlag John Wiley & Sons, New York (1979).

Beim Lösungsverfahren lässt sich die Molekülkomplexverbindung z.B. herstellen durch Lösen der beiden Komponenten (d.h. der Acylphosphinoxid-Verbindung und der α-Hydroxyketonverbindung), gegebenenfalls mittels Erwärmen, in geeigneten Lösungsmitteln oder auch Gemischen von Lösungsmitteln und Ausfällen der sich bildenden Molekülkomplexverbindungen entweder durch Zugabe eines Lösungsmittels, in welchem die entstandene Komplex-Verbindung schlechter löslich ist, oder durch sehr langsames Abkühlen der Lösung.
Zweckmässig wird beispielsweise eine bei höherer Temperatur gesättigte Lösung der Komponenten in einem homogen temperierten Gefäss sehr langsam auf eine tiefere Temperatur abgekühlt. Die Keimbildung der Kristalle kann dabei beispielsweise durch Reiben an der Gefässwand ausgelöst werden.
Es ist beispielsweise auch möglich einer gesättigten Lösung der Komponenten kontinuierlich das Lösungsmittel zu entziehen, beispielsweise durch Verdampfen, wobei dann die Bildung des Molekülkomplexkristalls eintritt.
Die Temperaturen, die bei der Herstellung der gesättigten Lösungen verwendet werden, sind abhängig vom eingesetzten Lösungsmittel bzw. Lösungsmittelgemisch und bewegen sich beispielsweise in einem Bereich von z.B. Raumtemperatur bis 150°C, insbesondere 50-100°C.
Vorzugsweise werden die beiden Komponenten bei der Herstellung der Molekülkomplexverbindungen im Molverhältnis 1:1 eingesetzt. Es ist jedoch auch möglich, die Komponenten in anderen Molverhältnissen einzusetzen, z.B. von 5:1 bis 1:5. Dabei kann dann beispielsweise eine Mischung aus Molekülkomplexverbindung und der im Überschuss zugegebenen Komponente entstehen.
Die Wahl des Lösungsmittels bei der Herstellung der Molekülkomplexverbindungen richtet sich nach dem jeweiligen Schmelzpunkt der Komponenten. Im vorliegenden Fall eignen sich als Lösungmittel für die verschiedenen Photoinitiatorverbindungen insbesondere aliphatische Kohlenwasserstoffe, wie z.B. Hexan, Pentan, Heptan, Octan und Isomerengemische der genannten Lösungsmittel. Möglich ist jedoch auch die Verwendung von aromatischen Kohlenwasserstoffen, wie beispielsweise Xylol oder Toluol usw..
Günstig ist die Verwendung von polaren Zusätzen, beispielsweise in der Grössenordnung von 1-30%, z.B. 1-20%, insbesondere 1-5%. Beispiele für solche Zusätze sind Essigester, Methylethylketon, Aceton, Methylisobutylketon oder Alkohole.

Auch die Verwendung von anderen polaren Lösungsmitteln, z.B linearen und cyclischen Ethern, wie Diethylether, Tetrahydrofuran oder Dioxan, ist denkbar.
Es können auch z.B. polare Lösungsmittel, wie z.B. Methylethylketon, verwendet werden und die entstandenen Komplexverbindungen mit Wasser ausgefällt werden. Diese Lösungsmittel können in reiner Form eingesetzt werden oder auch Wasser, beispielsweise als Azeotrop, enthalten. Das heisst, es sind auch rezyklierte Lösungsmittel, welche bei der Aufarbeitung im azeotropen Gemisch mit Wasser anfallen, geeignet.
Andere geeignete Lösungsmittel sind beispielsweise Siedegrenzebenzine mit ca. 3-10% Aromatenanteil. Diesen Lösungsmitteln können z.B. die oben beschriebenen polaren Lösungsmittel zugemischt werden. Weitere Beispiele für geeignete Lösungsmittelgemische sind Mischungen aus Isooctan und Ethylacetat, aber auch solche, die Wasser enthalten, wie oben bereits erwähnt, z.B. Methylethylketon und Wasser.
Die erhaltenen Molekülkomplexkristalle werden zweckmässig aus der Lösung durch übliche Trennungsmassnahmen, wie beispielsweise Filtration abgetrennt. Sind die verwendeten Lösungsmittel hochsiedende Lösungsmittel, so wird beim Abfiltrieren der ausgefallenen Molekülkomplexverbindungen mit einem niedrigsiedenden Lösungsmittel, wie beispielsweise Hexan nachgewaschen, um die Trocknung der Kristalle zu ermöglichen. Zweckmässig erfolgt die Trocknung der Kristalle bei leicht erhöhter Temperatur unter Anlegen eines Vakuums, insbesondere bei 40-50°C und ca. 50 mbar. Es kann auch zweckmässig sein, die Kristalle zu waschen, um sie von Unreinheiten zu befreien. Dies geschieht z.B. mit einem Lösungsmittel, in welchem die Kristalle schlecht löslich sind und welches mit der Mutterlauge mischbar ist.

Es ist z.B. auch möglich die Komplexverbindung herzustellen, indem direkt bei der Herstellung einer Komponente, vor deren Isolierung d.h. solange sie sich noch in Lösung befindet, die zweite Komponente zugegeben wird und die Komplexverbindung durch Zugabe eines entsprechenden Lösungsmittels ausgefällt wird. So kann z.B. bei der Herstellung der Acylphosphinoxidverbindung, nach dem Oxidationsschritt die Verbindung in ein geeigentes Lösungsmittel verbracht werden, die α-Hydroxyketonverbindung zugegeben und die Molekülkomplexverbindung ausgefällt werden.

Es ist beispielsweise auch möglich die erfindungsgemässen Molekülkomplexverbindungen durch Aufschmelzen der Acylphosphinoxidverbindungen und α-Hydroxyketonverbindungen und anschliessendes langsames Abkühlen der Schmelze zu erhalten. Dabei kann z.B. zunächst ein Gemisch der beiden Komponenten hergestellt und anschliessend aufgeschmolzen werden, es kann aber auch jede Komponenten für sich geschmolzen werden und die Verbindungen im geschmolzenen Zustand vermischt werden. Die Temperaturen sind abhängig von den Schmelzpunkten der jeweiligen Komponenten und liegen z.B. bei ca. 100°C bis 200°C. Es ist unter Umständen zweckmässig die Schmelze mit aus dem Lösungsmittelverfahren erhaltenen Kristallen der Molekülkomplexverbindungen anzuimpfen. Dies geschieht z.B. nach dem Abkühlen der Schmelze auf Raumtemperatur oder aber auch beim Schmelzpunkt der Molekülkomplexverbindung.
Solche Schmelzverfahren zur Herstellung von Kristallen sind dem Fachmann bekannt und z.B. In Ullmann's Encyclopedia of Industrial Chemistry, Vol. A 8, 121-127, Verlag Chemie, Weinheim-New York (1987) beschrieben.
Beim Abkühlen der Schmelze, insbesondere beim schnellen Abkühlen der Schmelze, können z.B. auch amorphe Modifikationen der erfindungsgemässen Molekülkomplexverbindungen erhalten werden. Zur Herstellung einiger Molekülkomplexverbindungen kann sich das schnelle Abkühlen als unzweckmässig erweisen, da sich der Komplex mit einigen Verbindungen nur langsam bildet. Beim schnellen Abkühlen ist auch die Wahrscheinlichkeit, dass Gemische zwischen der Komplexverbindung und einer ihrer Komponenten entstehen grösser.
Es ist z.b. auch denkbar, dass je nach Kristallisationsverfahren polymorphe Kristallformen der Molekülkomplexverbindungen oder beispielsweise Kristalle von Molekülkomplexverbindungen, welche "Gastmoleküle", z.B. Lösungsmittel enthalten, gebildet werden. Aber auch diese Gemische eignen sich als Initiatoren für die Photopolymerisation.

Die Molekülkomplexe sind in der Regel über Wasserstoffbrücken zwischen den H-Atomen der OH-Gruppe des Hydroxyketons und dem am P-Atom gebundenen Sauerstoffatom der Phosphinoxidverbindung assoziiert.

Bevorzugt sind Molekülkomplexverbindungen, worin die Mono-, Bis- oder Trisacylphosphinoxid-Verbindung eine Verbindung der Formel I 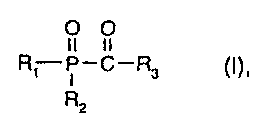 worin
R₁ und R₂ unabhängig voneinander C₁-C₁₈-Alkyl, durch ein oder mehrere O-Atome unterbrochenes C₂-C₁₈-Alkyl, durch Phenyl substituiertes C₁-C₄-Alkyl, C₂-C₁₈-Alkenyl, unsubstituiertes oder ein- bis fünfmal mit Halogen, Hydroxy, C₁-C₈-Alkyl und/oder C₁-C₈-Alkoxy substituiertes Phenyl, unsubstituiertes oder ein- bis fünfmal mit Halogen, Hydroxy, C₁-C₈-Alkyl und/oder C₁-C₈-Alkoxy substituiertes Naphthyl, unsubstituiertes oder ein- bis fünffach mit Halogen, Hydroxy, C₁-C₈-Alkyl und/oder C₁-C₈-Alkoxy substituiertes Biphenyl, C₃-C₁₂-Cycloalkyl, einen O-, S- oder N-haltigen 5- oder 6- gliedrigen heterocyclischen Ring oder eine Gruppe COR₃ darstellen; oder
R₁ -OR₄ oder eine Gruppe 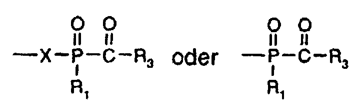 ist; oder
R₁ und R₂ zusammen C₄-C₈-Alkylen bedeuten und mit dem P-Atom, an welches sie gebunden sind eine Ringstruktur bilden;
R₃ C₁-C₁₈-Alkyl, C₃-C₁₂-Cycloalkyl, C₂-C₁₈-Alkenyl, unsubstituiertes oder ein- bis vierfach mit C₁-C₈-Alkyl, C₁-C₈-Alkoxy, C₁-C₈-Alkylthio und/oder Halogen substituiertes Phenyl, Naphthyl oder Biphenyl, einen O-, S- oder N-haltigen 5- oder 6-gliedrigen heterocyclischen Ring oder eine Gruppe 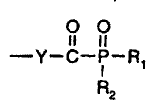 darstellt;
R₄ C₁-C₈-Alkyl, Phenyl, Naphthyl, Phenyl-C₁-C₈-alkyl bedeutet;
Y Phenylen, C₁-C₁₂-Alkylen, Cyclopentylen oder Cyclohexylen ist;
X C₁-C₁₈-Alkylen, ein- oder mehrmals durch -O-, -S-, -NR₅-, 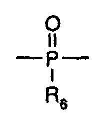 oder -SO₂- unterbrochenes C₂-C₁₈-Alkylen, durch Cl, F, C₁-C₄-Alkoxy, COOR₇, Phenyl, Phenyl-C₁-C₄-alkyl, Naphthyl-C₁-C₄-alkyl, C₁-C₄-Alkylphenyl, C₁-C₄-Alkylnaphthyl, Phenyl-C₁-C₄-alkoxy, Naphthyl-C₁-C₄-alkoxy, C₁-C₄-Alkoxy-C₁-C₄alkoxy und/oder CN substituiertes C₁-C₆-Alkylen ist, oder
X C₁-C₈-Alkylen, das durch einen oder zwei Reste der Formel A 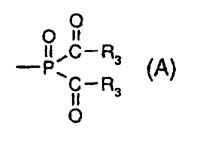 substituiert ist, oder
X eine Gruppe der Formel A₁-A₉ 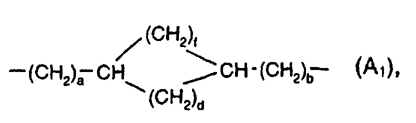 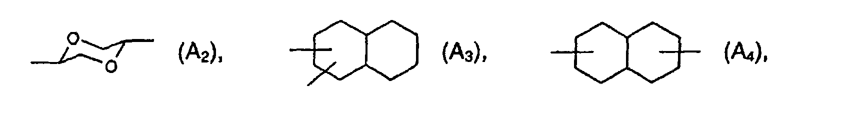 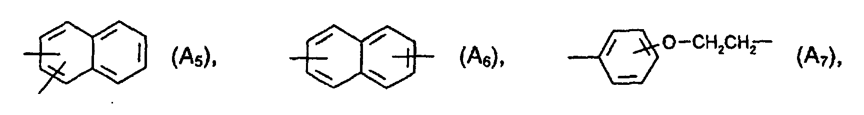 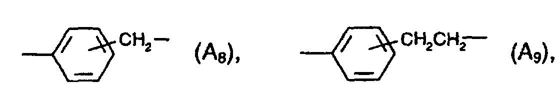 bedeutet, wobei
a und b unabhängig voneinander 0 oder 1 sind und die Summe von d und f eine Zahl von 3 bis 8 ist, wobei weder d noch f 0 sind, oder
X eine Gruppe -CH₂-CH=CH-CH₂- oder -CH₂-C≡C-CH₂- darstellt, oder unsubstituiertes oder ein- bis dreifach mit Cl, F, C₁-C₄-Alkyl und/oder C₁-C₄-Alkoxy substituiertes Phenylen, Xylylen, 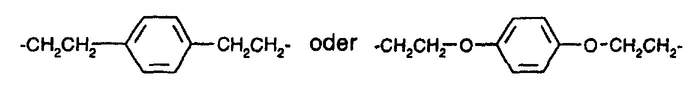 ist oder
X eine Gruppe der Formel A₁₀-A₁₃ 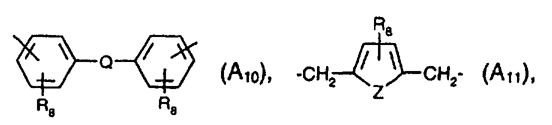 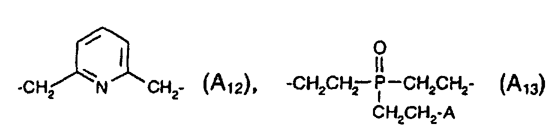 darstellt;
Q eine Einfachbindung, CR₉R₁₀, -O-, -S-, -NR₅-, -SO₂-, -(CH₂)ₚ- oder -CH=CH- bedeutet;
p eine Zahl von 2-12 ist;
Z O oder S bedeutet;
R₅ Wasserstoff, C₁-C₁₂-Alkyl oder Phenyl ist;
R₆ C₁-C₄-Alkyl oder Phenyl bedeutet;
R₇ C₁-C₁₂-Alkyl, ein- oder mehrfach durch -O- unterbrochenes C₂-C₁₈-Alkyl, Benzyl, Phenyl, Cyclopentyl oder Cyclohexyl bedeutet;
R₈ Wasserstoff, C₁-C₄-Alkyl, C₁-C₄-Alkoxy oder Halogen bedeutet;
R₉ Wasserstoff oder C₁-C₄-Alkyl ist;
R₁₀ Wasserstoff, Methyl oder Ethyl bedeutet, ist.

C₁-C₁₈-Alkyl kann linear oder verzweigt sein und bedeutet beispielsweise Methyl, Ethyl, Propyl, Isopropyl, Butyl, Isobutyl, sec.-Butyl, tert.-Butyl, Pentyl, Isopentyl, Hexyl, Heptyl, Octyl, Nonyl, Decyl, Dodecyl oder Octadecyl. Bevorzugt sind C₁-C₁₂-, z.B. C₁-C₈- oder C₁-C₆-, insbesondere C₁-C₄-Alkyl.
C₁-C₁₂-Alkyl, C₁-C₈-Alkyl und C₁-C₄-Alkyl können die gleichen Bedeutungen haben wie oben angegeben, bis zur entsprechenden Anzahl der C-Atome.

Durch ein oder mehrere O-Atome unterbrochenes C₂-C₁₈-Alkyl ist beispielsweise 1 bis 5, z.B. 1 bis 3 oder 1 oder 2 mal durch -O- unterbrochen. Es ergeben sich z.B. Struktureinheiten wie -O(CH₂)₂OH, -O(CH₂)₂OCH₃, -O(CH₂CH₂O)₂CH₂CH₃, -CH₂-O-CH₃, -CH₂CH₂-O-CH₂CH₃, -[CH₂CH₂O]_{y}-CH₃, mit y = 1 bis 5, -(CH₂CH₂O)₅CH₂CH₃, -CH₂-CH(CH₃)-O-CH₂-CH₂CH₃ oder -CH₂-CH(CH₃)-O-CH₂-CH₃.

Durch Phenyl substituiertes C₁-C₄-Alkyl bedeutet z.B. Benzyl, 2-Phenylethyl, 3-Phenylpropyl, α-Methylbenzyl oder α,α-Dimethylbenzyl, insbesondere Benzyl.

Phenyl-C₁-C₈-alkyl bedeutet z.B. Benzyl, Phenylethyl, α-Methylbenzyl, Phenylpentyl, Phenylhexyl, Phenyloctyl oder α,α-Dimethylbenzyl, insbesondere Benzyl. Phenyl-C₁-C₄-alkyl hat die oben angegebenen Bedeutungen bis zur entsprechenden Anzahl der C-Atome. Bevorzugt ist Phenyl-C₁-C₄-alkyl, insbesondere Phenyl-C₁-C₂-alkyl.

C₂-C₁₈-Alkenyl kann linear oder verzweigt sein und es können mehr als eine ungesättigte Bindung im Molekül vorhanden sein. Beispiele sind Vinyl, Allyl, Methylvinyl, Butenyl, Butadienyl, Pentenyl, Hexenyl, Heptenyl, Octenyl, Nonenyl, Decenyl, Dodecenyl oder Octadecenyl.

C₁-C₈-Alkoxy kann linear oder verzweigt sein und bedeutet beispielsweise Methoxy, Ethoxy, Propoxy, Isopropoxy, Butoxy, Isobutoxy, sec.-Butoxy, tert.-Butoxy, Pentoxy, Isopentoxy, Hexyloxy, Heptyloxy oder Octyloxy. Bevorzugt sind z.B. C₁-C₆- oder insbesondere C₁-C₄-Alkoxy.

C₁-C₆-Alkoxy und C₁-C₄-Alkoxy können die gleichen Bedeutungen haben wie oben angegeben, bis zur entsprechenden Anzahl der C-Atome.

C₁-C₄-Alkoxy-C₁-C₄-alkoxy bedeutet beispielsweise Methoxyethoxy, Methoxypropoxy, Methoxybutoxy, Ethoxymethoxy, Ethoxyethoxy, Ethoxypropoxy, Ethoxybutoxy, Propoxymethoxy, Propoxyethoxy, Propoxypropoxy, Propoxybutoxy, Butoxymethoxy, Butoxyethoxy, Butoxypropoxy oder Butoxybutoxy, insbesondere Methoxyethoxy und Ethoxyethoxy.

C₁-C₈-Alkylthio kann linear oder verzweigt sein und ist beispielsweise Methylthio, Ethylthio, Propylthio, Isopropylthio, Butylthio, tert-Butylthio, Hexylthio oder Octylthio, insbesondere Methylthio.

Halogen steht beispielsweise für Chlor, Brom und lod, insbesondere für Chlor.

Substituiertes Phenyl ist ein- bis fünffach, z.B. ein-, zwei- oder dreifach, insbesondere einoder zweifach am Phenylring substituiert. Die Substitution erfolgt z.B. in 2-, 3-, 4-, 5-, 2,4-, 2,5-, 2,6-, 3,4-, 3,5-, 2,4,6- oder 3,4,5-Stellung des Phenylrings. C₁-C₈-Alkyl, C₁-C₄-Alkyl-, C₁-C₈-Alkoxy-, C₁-C₈Alkylthio- und C₁-C₄-Alkoxysubstituenten können die oben angegebenen Bedeutungen haben. Beispiele für substituiertes Phenyl sind Tolyl, Xylyl, 4-Methoxyphenyl, 2,4-, 2,5-Dimethoxyphenyl, Ethylphenyl, 4-Alkoxy-2-methylphenyl.

Beispiele für C₃-C₁₂-Cycloalkyl sind Cyclopentyl, Cyclohexyl, Cycloheptyl, Cyclooctyl, Cyclononyl, Cyclodecyl oder Cyclododecyl, bevorzugt sind Cyclopentyl und Cyclohexyl.

Als O-, S- oder N-haltiger 5- oder 6-gliedriger heterocyclischer Ring bedeuten R₁ und R₂ z.B. Furyl, Thienyl, Pyrrolyl, Oxinyl, Dioxinyl oder Pyridyl.

Wenn R₁ und R₂ zusammen C₄-C₈-Alkylen bedeuten und mit dem P-Atom, an welches sie gebunden sind, eine Ringstruktur bilden, so können dies nicht nur einfache Ringe sein, sondern auch überbrückte Ringe, wie z.B. .

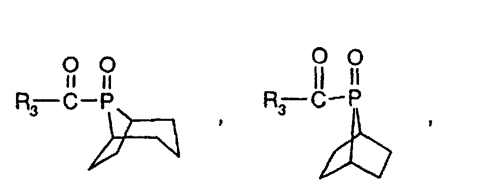

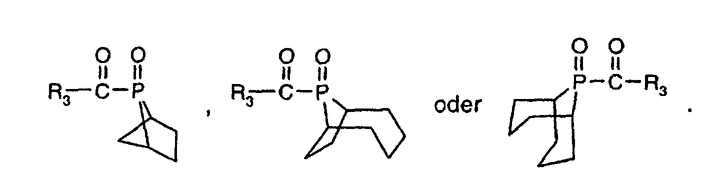

X als C₁-C₁₈-Alkylen ist lineares oder verweigtes Alkylen wie z.B. Methylen, Ethylen, Propylen, Isopropylen, n-Butylen, sec-Butylen, iso-Butylen, tert-Butylen, Pentylen, Hexylen, Heptylen, Octylen, Nonylen, Decylen, Dodecylen, Tetradecylen, Heptadecylen oder Octadecylen. Insbesondere ist X C₁-C₁₂-Alkylen, z.B. Ethylen, Decylen, 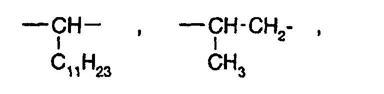 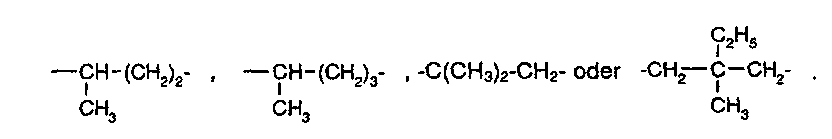 Ist X als C₂-C₁₈-Alkylen unterbrochen mit -O-, -S-, -NR₅-, 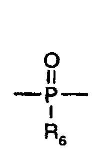 oder -SO₂- ergeben sich z.B. Struktureinheiten wie -CH₂-O-CH₂-, -CH₂CH₂-O-CH₂CH₂-, -[CH₂CH₂O]_{y}-, mit y = 1-9, -(CH₂CH₂O)₇CH₂CH₂-, -CH₂-CH(CH₃)-O-CH₂-CH(CH₃)-, -CH₂-S-CH₂-, -CH₂CH₂-S-CH₂CH₂-, -CH₂CH₂CH₂-S-CH₂CH₂CH₂-, -(CH₂)₃-S-(CH₂)₃-S-(CH₂)₃-, -CH₂-(NR₅)-CH₂-, -CH₂CH₂-(NR₅)-CH₂CH₂-, -CH₂-(P(O)R₆)-CH₂-, -CH₂CH₂-(P(O)R₆)-CH₂CH₂-, 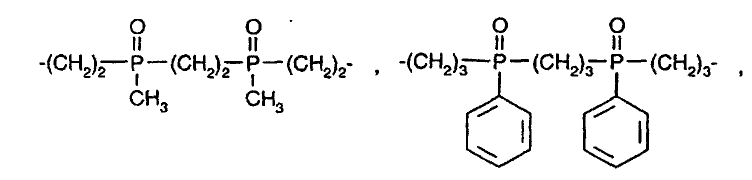 -CH₂-SO₂-CH₂- oder -CH₂CH₂-SO₂-CH₂CH₂-.

Beispiele für C₁-C₈-Alkylen, welches mit einem oder zwei Resten der Formel A substituiert ist, sind 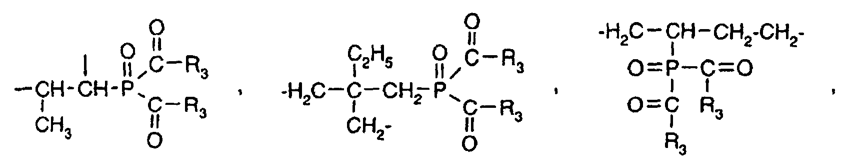 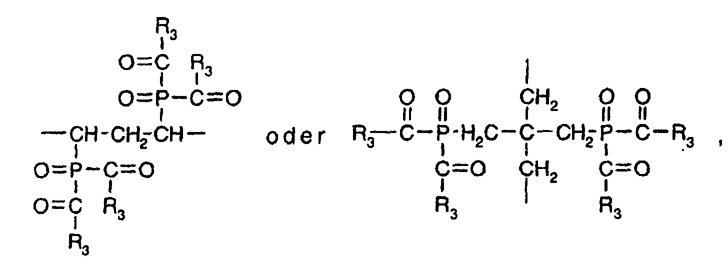 wobei R₃ wie oben definiert ist.

Naphthyl-C₁-C₄-alkyl ist z.B. Naphthylmethyl, Naphthylethyl, Naphthyl-(1-methyl)eth-1-yl oder Naphthyl-(1,1-dimethyl)eth-1-yl, insbesondere Naphthylmethyl.

C₁-C₄-Alkylphenyl ist beispielsweise Tolyl, Xylyl, Mesityl, Ethylphenyl, Diethylphenyl, vorzugsweise Tolyl oder Mesityl. C₁-C₄-Alkylnaphthyl ist mit Methyl, Ethyl und/oder Propyl oder Butyl substituiertes Naphthyl.

Unter Phenyl-C₁-C₄-alkoxy ist beispielsweise Benzyloxy, Phenylethyloxy, α-Methylbenzyloxy oder α,α-Dimethylbenzyloxy, insbesondere Benzyloxy zu verstehen. Naphthyl-C₁-C₄-alkoxy ist z.B. Naphthylmethyloxy oder Naphthylethyloxy.

Beispiele für Gruppen der Formel A₁, worin die Summe von d und f 3 bis 8 ist, sind: 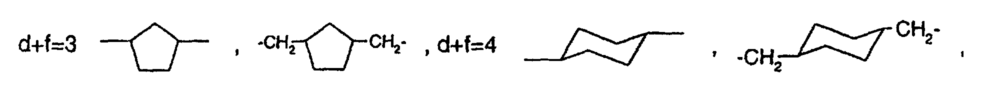 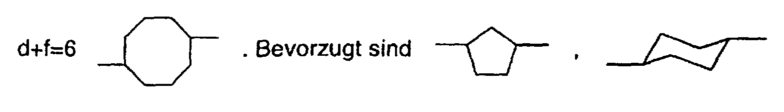 und

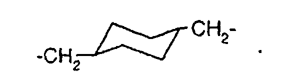

Bevorzugt als Gruppe der Formel A₁₀ sind

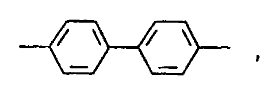

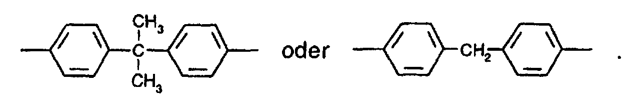

Die Herstellung von Acylphosphinoxidverbindungen der Formel ist dem Fachmann bekannt und beispielsweise beschrieben in der EP-A 7 508, den US Patenten 5,218,009; 4,737,593; 4,792,632, in der GB-A 2 259 704 und in der WO-A 96/7662.

Bevorzugt sind Verbindungen der Formel I, worin R₁ und R₂ unabhängig voneinander C₁-C₁₂-Alkyl, Benzyl, unsubstituiertes oder ein- bis vierfach mit Halogen, insbesondere Cl, C₁-C₈-Alkyl und/oder C₁-C₈-Alkoxy substituiertes Phenyl, Cyclohexyl oder COR₃ bedeuten, oder R₁ für OR₄ oder 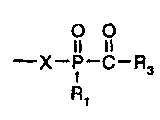 steht;
R₃ unsubstituiertes oder ein- bis vierfach mit C₁-C₈-Alkyl, insbesondere C₁-C₄-Alkyl, C₁-C₈-Alkoxy, C₁-C₈-Alkylthio, insbesondere C₁-C₄-Alkylthio, und/oder Halogen, insbesondere Chlor, substituiertes Phenyl oder eine Gruppe 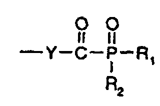 ist;
R₄ C₁-C₈-Alkyl, insbesondere C₁-C₄-Alkyl, Phenyl oder Benzyl ist;
X C₁-C₁₈-Alkylen, insbesondere C₆-C₁₀-Alkylen, oder eine Gruppe 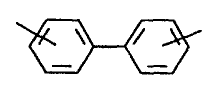 ist;
und Y Phenylen, C₂-C₁₂-Alkylen oder Cyclohexylen darstellt.

Weitere interessante Verbindungen der Formel I sind solche, worin R₁ und R₂ unabhängig voneinander C₁-C₈-Alkyl, unsubstituiertes oder ein- bis viermal durch C₁-C₄-Alkyl und/oder C₁-C₈-Alkoxy substituiertes Phenyl, cyclohexyl oder eine Gruppe COR₃ bedeuten, oder R₁ für eine Gruppe 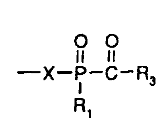 oder -OR₄ steht; R₃ unsubstituiertes oder ein- bis viermal durch Methyl und/oder Methoxy substituiertes Phenyl ist; R₄ Methyl, Ethyl oder Phenyl darstellt; und X C₆-C₁₀-Alkylen oder 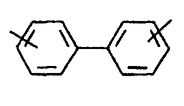 ist.

Hervorzuheben sind Verbindungen der Formel I, worin R₁ für COR₃ steht, R₂ C₁-C₁₈-Alkyl bedeutet und R₃ Phenyl, welches zwei-oder dreifach mit C₁-C₄-Alkyl oder C₁-C₄-Alkoxy substituiert ist bedeutet.

Insbesondere sind auch solche Verbindungen der Formel I interessant, worin R₁ für COR₃ steht, R₂ unsubstituiertes oder mit C₁-C₄-Alkyl oder/und C₁-C₄-Alkoxy substituiertes Phenyl bedeutet und R₃ Phenyl, welches zwei-oder dreifach mit C₁-C₄-Alkyl oder C₁-C₄-Alkoxy substituiert ist bedeutet.

Besonders interessant sind Verbindungen der Formel I, wenn die Substitution bei R₃ als Phenyl in 2,6- oder 2,4,6-Stellung erfolgt.

Beispiele für zur Herstellung der Molekülkomplexverbindungen geeignete Verbindungen der Formel I sind
Bis(2,6-dimethoxybenzoyl)-phenyl-phosphinoxid,
Bis(2,6-dimethoxybenzoyl)-(2,4,4-trimethyl-pentyl)-phosphinoxid,
Bis(2,6-dimethoxybenzoyl)-n-butyl-phosphinoxid,
Bis(2,6-dimethoxybenzoyl)-(2-methyl-prop-1-yl)-phosphinoxid,
Bis(2,6-dimethoxybenzoyl)-(1 -methyl-prop-1 -yl)-phosphinoxid,
Bis(2,6-dimethoxybenzoyl)-t-butyl-phosphinoxid,
Bis(2,6-dimethoxybenzoyl)-cyclohexyl-phosphinoxid,
Bis(2,6-dimethoxybenzoyl)-octyl-phosphinoxid,
Bis(2-methoxybenzoyl)-(2-methyl-prop-1-yl)-phosphinoxid,
Bis(2-methoxybenzoyl)-(1-methyl-prop-1 -yl)-phosphinoxid,
Bis(2,6-diethoxybenzoyl)-(2-methyl-prop-1-yl)-phosphinoxid,
Bis(2,6-diethoxybenzoyl)-(1-methyl-prop-1-yl)-phosphinoxid,
Bis(2,6-dibutoxybenzoyl)-(2-methyl-prop-1-yl)-phosphinoxid,
Bis(2,4-dimethoxybenzoyl)-(2-methyl-prop-1-yl)-phosphinoxid,
Bis(2,4,6-trimethylbenzoyl)-phenyl-phosphinoxid,
2,4,6-Trimethylbenzoyl-diphenylphosphinoxid,
Bis(2,4,6-trimethylbenzoyl)-(2,4-dipentoxyphenyl)-phosphinoxid,
Bis(2,6-dimethoxybenzoyl)-benzylphosphinoxid,
Bis(2,6-dimethoxybenzoyl)-2-phenylpropylphosphinoxid,
Bis(2,6-dimethoxybenzoyl)-2-phenylethylphosphinoxid,
Bis(2,6-dimethoxybenzoyl)-benzylphosphinoxid,
Bis(2,6-dimethoxybenzoyl)-2-phenylpropylphosphinoxid,
Bis(2,6-dimethoxybenzoyl)-2-phenylethylphosphinoxid,
2,6-Dimethoxybenzoyl-benzyl-butylphosphinoxid,
2,6-Dimethoxybenzoyl-benzyl-octylphosphinoxid,
Bis(2,4,6-trimethylbenzoyl)-isobutylphosphinoxid,
2,6-Dimethoxybenzoyl-2,4,6-trimethylbenzoyl-n-butyl-phosphinoxid.

Die α-Hydroxyketonverbindungen sind insbesondere Verbindungen der Formel II 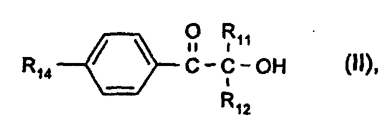 worin
R₁₁ und R₁₂ unabhängig voneinander Wasserstoff, C₁-C₆-Alkyl, Phenyl, C₁-C₆-Alkoxy, OSiR₁₆(R₁₇)₂ oder -O(CH₂CH₂O)_{q}-C₁-C₆-alkyl bedeuten, oder
R₁₁ und R₁₂ zusammen mit dem Kohlenstoff-Atom, an welches sie gebunden sind, einen Cyclohexylring bilden;
q eine Zahl von 1 bis 20 ist;
R₁₄ Wasserstoff, C₁-C₁₈-Alkyl, C₁-C₁₈-Alkoxy, -OCH₂CH₂-OR₁₅, eine Gruppe CH₂=C(CH₃)- oder 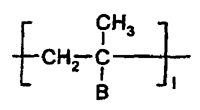 darstellt;
1 für eine Zahl von 2 bis 10 steht;
B den Rest 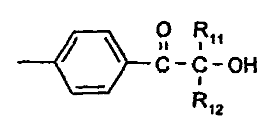 darstellt;
R₁₅ Wasserstoff, 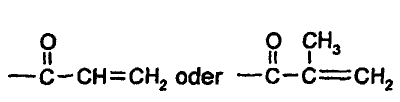 darstellt und
R₁₆ und R₁₇ unabhängig voneinander C₁-C₈-Alkyl oder Phenyl sind; ist.

Beispiele für C₁-C₁₈-Alkyl, C₁-C₆-Alkyl, C₁-C₄-Alkyl, C₁-C₁₈-Alkoxy und C₁-C₆-Alkoxy sind oben angegeben.

Die Herstellung der α-Hydroxyketonverbindungen der Formel II ist dem Fachmann geläufig und beispielsweise beschrieben in den US Patenten 4,347,111 und 4,672,079 sowie in der EP-A 3002.

Interessante Verbindungen der Formel II sind solche, worin R₁₁ und R₁₂ unabhängig voneinander Wasserstoff, C₁-C₆-Alkyl oder Phenyl bedeuten oder R₁₁ und R₁₂ zusammen mit dem Kohlenstoffatom, an welches sie gebunden sind, einen Cyclohexylring bilden; und R₁₄ Wasserstoff, C₁-C₁₂-Alkyl, C₁-C₁₂-Alkoxy, insbesondere C₁-C₄-Alkoxy, -OCH₂CH₂OR₁₅, eine Gruppe CH₂=C(CH₃)- oder 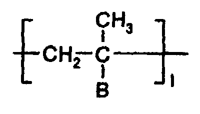 darstellt.

Bevorzugt sind die Verbindungen der Formel II, worin R₁₁ und R₁₂ unabhängig voneinander Methyl oder Ethyl bedeuten oder R₁₁ und R₁₂ zusammen mit dem Kohlenstoffatom, an welches sie gebunden sind, einen Cyclohexylring bilden; und R₁₄ Wasserstoff, C₁-C₄-Alkyl, C₁-C₄-Alkoxy oder -OCH₂CH₂OH bedeutet.

Beispiele für zur Herstellung der Molekülkomplexverbindungen geeignete Verbindungen der Formel II sind
α-Hydroxy-cyclohexylphenyl-keton,
2-Hydroxy-2-methyl-1-phenyl-propanon,
2-Hydroxy-2-methyl-1(4-isopropylphenyl)-propanon,
2-Hydroxy-2-methyl-1(4-dodecylphenyl)-propanon,
2-Hydroxy-2-methyl-1[(2-hydroxy-ethoxy)-phenyl]-propanon.

Bevorzugte Molekülkomplexverbindungen sind solche, aus einer Mono- oder Bisacylphosphinoxid-Verbindung der Formel I 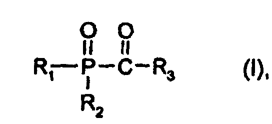 worin
R₁ und R₂ unabhängig voneinander C₁-C₁₂-Alkyl, unsubstituiertes oder ein- oder zweifach mit C₁-C₈-Alkyl, C₁-C₈-Alkoxy substituiertes Phenyl oder COR₃ bedeuten;
R₃ für einen Rest 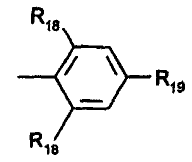 steht;
R₁₈ C₁-C₄-Alkyl, insbesondere Methyl, oder C₁-C₄-Alkoxy, insbesondere Methoxy, ist; und R₁₉ für Wasserstoff oder C₁-C₄-Alkyl, insbesondere Methyl, steht;
und einer α-Hydroxyketonverbindung der Formel II 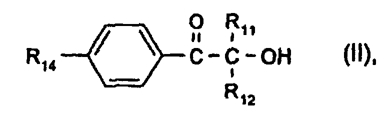 worin
R₁₁ und R₁₂ unabhängig voneinander C₁-C₄-Alkyl bedeuten oder R₁₁ und R₁₂ zusammen mit dem Kohlenstoffatom, an welches sie gebunden sind, einen Cyclohexylring bilden; und
R₁₄ für Wasserstoff steht.

Bevorzugt sind weiterhin Molekülkomplexverbindungen, worin die Acylphosphinoxid-Verbindung Bis(2,6-dimethoxybenzoyl)-(2,4,4-trimethyl-pentyl)-phosphinoxid oder 2,4,6-Trimethylbenzoyl-diphenylphosphinoxid ist und die α-Hydroxyketon-Verbindung eine Verbindung der Formel II ist, worin R₁₁ und R₁₂ für C₁-C₄-Alkyl stehen oder R₁₁ und R₁₂ zusammen mit dem C-Atom, an welches sie gebunden sind, einen Cyclohexylring bilden, und R₁₄ Wasserstoff ist.

Weiterhin interessant sind Molekülkomplexverbindungen, worin die Acylphosphinoxid-Verbindung Bis(2,6-dimethoxybenzoyl)-(2,4,4-trimethyl-pentyl)-phosphinoxid, Bis(2,4,6-trimethylbenzoyl)-(2,4-dihexyloxyphenyl)-phosphinoxid, Bis(2,4,6-trimethylbenzoyl)-(4-ethoxyphenyl)-phosphinoxid oder 2,4,6-Trimethylbenzoyl-diphenylphosphinoxid ist und die α-Hydroxyketon-Verbindung α-Hydroxy-cyclohexyl-phenyl-keton oder 2-Hydroxy-2-methyl-1-phenyl-propan-1-on ist.

Insbesondere interessant sind Molekülkomplexverbindungen aus
Bis(2,6-dimethoxybenzoyl)-(2,4,4-trimethyl-pentyl)-phosphinoxid und 2-Hydroxy-2-methyl-1-phenyl-propanon;
Bis(2,6-dimethoxybenzoyl)-(2,4,4-trimethyl-pentyl)-phosphinoxid und a-Hydroxy-cyclohexyl-phenyl-keton;
Bis(2,4,6-trimethylbenzoyl)-phenyl-phosphinoxid und α-Hydroxy-cyclohexyl-phenyl-keton;
Bis(2,4,6-trimethylbenzoyl)-phenyl-phosphinoxid und 2-Hydroxy-2-methyl-1-phenylpropanon;
2,4,6-Trimethylbenzoyl-diphenylphosphinoxid und α-Hydroxy-cyclohexyl-phenyl-keton;
2,4,6-Trimethylbenzoyl-diphenylphosphinoxid und 2-Hydroxy-2-methyl-1-phenyl-propanon;
Bis(2,4,6-trimethylbenzoyl)-(4-ethoxyphenyl)-phosphinoxid und α-Hydroxy-cyclohexyl-phenyl-keton;
Bis(2,4,6-trimethylbenzoyl)-(2,4-dipentoxyphenyl)-phosphinoxid und α-Hydroxy-cyclohexylphenyl-keton;
Bis(2,4,6-trimethylbenzoyl)-(2,4-dihexyloxyphenyl)-phosphinoxid und α-Hydroxy-cyclohexylphenyl-keton;
Bis(2,4,6-trimethylbenzoyl)-(2,4-dipentoxyphenyl)-phosphinoxid und 2-Hydroxy-2-methyl-1-phenyl-propanon;
Bis(2,4,6-trimethylbenzoyl)-(2-methyl-propyl)-phosphinoxid und α-Hydroxy-cyclohexyl-phenyl-keton;
Bis(2,4,6-trimethylbenzoyl)-(2-methyl-propyl)-phosphinoxid und 2-Hydroxy-2-methyl-1-phenyl-propanon;
Bis(2,6-dimethoxybenzoyl)-(2-methyl-propyl)-phosphinoxid und 2-Hydroxy-2-methyl-1-phenyl-propanon;
Bis(2,6-dimethoxybenzoyl)-(2-methyl-propyl)-phosphinoxid und α-Hydroxy-cyclohexyl-phenyl-keton.

Erfindungsgemäss können die Molekülkomplexverbindungen als Photoinitiatoren für die Photopolymerisation von ethylenisch ungesättigten Verbindungen bzw. Gemischen, die solche Verbindungen enthalten, verwendet werden.
Diese Verwendung kann auch in Kombination mit einem anderen Photoinitiator und/oder anderen Additiven erfolgen.

Die Erfindung betrifft daher auch photopolymerisierbare Zusammensetzungen, enthaltend
(a) mindestens eine ethylenisch ungesättigte photopolymerisierbare Verbindung und
(b) als Photoinitiator mindestens eine Molekülkomplexverbindung aus einer Mono-, Bisoder Trisacylphosphinoxid-Verbindung mit einer α-Hydroxyketon-Verbindung,
wobei die Zusammensetzung neben der Komponente (b) noch andere Photoinitiatoren und/oder andere Additive enthalten kann.

Die ungesättigten Verbindungen können eine oder mehrere olefinische Doppelbindungen enthalten. Sie können niedermolekular (monomer) oder höhermolekular (oligomer) sein. Beispiele für Monomere mit einer Doppelbindung sind Alkyl- oder Hydroxyalkyl-acrylate oder -methacrylate, wie z.B. Methyl-, Ethyl-, Butyl-, 2-Ethylhexyl- oder 2-Hydroxyethylacrylat, Isobornylacrylat, Methyl- oder Ethylmethacrylat. Interessant sind auch Silicon-acrylate. Weitere Beispiele sind Acrylnitril, Acrylamid, Methacrylamid, N-substituierte (Meth)acrylamide, Vinylester wie Vinylacetat, Vinylether wie Isobutylvinylether, Styrol, Alkyl- und Halogenstyrole, N-Vinylpyrrolidon, Vinylchlorid oder Vinylidenchlorid.

Beispiele für Monomere mit mehreren Doppelbindungen sind Ethylenglykol-, Propylenglykol-, Neopentylglykol-, Hexamethylenglykol- oder Bisphenol-A-diacrylat, 4,4'-Bis(2-acryloyloxyethoxy)-diphenylpropan, Trimethylolpropan-triacrylat, Pentaerythrittriacrylat oder -tetraacrylat, Vinylacrylat, Divinylbenzol, Divinylsuccinat, Diallylphthalat, Triallylphosphat, Triallylisocyanurat oder Tris-(2-acryloylethyl)isocyanurat.

Beispiele für höhermolekulare (oligomere) mehrfach ungesättigte Verbindungen sind acrylierte Epoxidharze, acrylierte oder Vinylether- oder Epoxy-Gruppen enthaltende Polyester, Polyurethane und Polyether. Weitere Beispiele für ungesättigte Oligomere sind ungesättigte Polyesterharze, die meist aus Maleinsäure, Phthalsäure und einem oder mehreren Diolen hergestellt werden und Molekulargewichte von etwa 500 bis 3000 besitzen. Daneben können auch Vinylether-Monomere und -Oligomere, sowie maleat-terminierte Oligomere mit Polyester-, Polyurethan-, Polyether-, Polyvinylether- und Epoxidhauptketten eingesetzt werden. Insbesondere Kombinationen von Vinylethergruppen tragenden Oligomeren und Polymeren, wie sie in der WO 90/01512 beschrieben sind, sind gut geeignet. Aber auch Copolymere aus Vinylether und Maleinsäure funktionalisierten Monomeren kommen in Frage. Solche ungesättigten Oligomere kann man auch als Prepolymere bezeichnen.

Besonders geeignet sind z.B. Ester von ethylenisch ungesättigten Carbonsäuren und Polyolen oder Polyepoxiden, und Polymere mit ethylenisch ungesättigten Gruppen in der Kette oder in Seitengruppen, wie z. B. ungesättigte Polyester, Polyamide und Polyurethane und Copolymere hiervon, Polybutadien und Butadien-Copolymere, Polyisopren und Isopren-Copolymere, Polymere und Copolymere mit (Meth)Acrylgruppen in Seitenketten, sowie Mischungen von einem oder mehreren solcher Polymerer.

Beispiele für ungesättigte Carbonsäuren sind Acrylsäure, Methacrylsäure, Crotonsäure, Itaconsäure, Zimtsäure, ungesättigte Fettsäuren wie Linolensäure oder Oelsäure. Bevorzugt sind Acryl- und Methacrylsäure.

Als Polyole sind aromatische und besonders aliphatische und cycloaliphatische Polyole geeignet. Beispiele für aromatische Polyole sind Hydrochinon, 4,4'-Dihydroxydiphenyl, 2,2-Di(4-hydroxyphenyl)-propan, sowie Novolake und Resole. Beispiele für Polyepoxide sind solche auf der Basis der genannten Polyole, besonders der aromatischen Polyole und Epichlorhydrin. Ferner sind auch Polymere und Copolymere, die Hydroxylgruppen in der Polymerkette oder in Seitengruppen enthalten, wie z.B. Polyvinylalkohol und Copolymere davon oder Polymethacrylsäurehydroxyalkylester oder Copolymere davon, als Polyole geeignet. Weitere geeignete Polyole sind Oligoester mit Hydroxylendgruppen.

Beispiele für aliphatische und cycloaliphatische Polyole sind Alkylendiole mit bevorzugt 2 bis 12 C-Atomen, wie Ethylenglykol, 1,2- oder 1,3-Propandiol, 1,2-, 1,3- oder 1,4-Butandiol, Pentandiol, Hexandiol,Octandiol, Dodecandiol, Diethylenglykol, Triethylenglykol, Polyethylenglykole mit Molekulargewichten von bevorzugt 200 bis 1500, 1,3-Cyclopentandiol, 1,2-, 1,3- oder 1,4-Cyclohexandiol, 1,4-Dihydroxymethylcyclohexan, Glycerin, Tris-(β-hydroxyethyl)amin, Trimethylolethan, Trimethylolpropan, Pentaerythrit, Dipentaerythrit und Sorbit.

Die Polyole können teilweise oder vollständig mit einer oder verschiedenen ungesättigten Carbonsäuren verestert sein, wobei in Teilestern die freien Hydroxylgruppen modifiziert, z.B. verethert oder mit anderen Carbonsäuren verestert sein können.

Beispiele für Ester sind:
Trimethylolpropantriacrylat, Trimethylolethantriacrylat, Trimethylolpropantrimethacrylat, Trimethylolethantrimethacrylat, Tetramethylenglykoldimethacrylat, Triethytenglykoldimethacrylat, Tetraethylenglykoldiacrylat, Pentaerythritdiacrylat, Pentaerythrittriacrylat, Pentaerythrittetraacrylat, Dipentaerythritdiacrylat, Dipentaerythrittriacrylat, Dipentaerythrit-tetraacrylat, Dipentaerythritpentaacrylat, Dipentaerythrithexaacrylat, Tripentaerythritocta-acrylat, Pentaerythritdimethacrylat, Pentaerythrittrimethacrylat, Dipentaerythritdimethacrylat, Dipentaerythrittetramethacrylat, Tripentaerythritoctamethacrylat, Pentaerythritdiitaconat, Dipentaerythrittrisitaconat, Dipentaerythritpentaitaconat, Dipentaerythrithexaitaconat, Ethylenglykoldiacrylat, 1,3-Butandioldiacrylat, 1,3-Butandioldimethacrylat, 1,4-Butandioldi-itaconat, Sorbittriacrylat, Sorbittetraacrylat, Pentaerythrit-modifiziert-triacrylat, Sorbittetra-methacrylat, Sorbitpentaacrylat, Sorbithexaacrylat, Oligoesteracrylate und -methacrylate, Glycerindi- und -triacrylat, 1,4-Cyclohexandiacrylat, Bisacrylate und Bismethacrylate von Polyethylenglykol mit Molekulargewicht von 200 bis 1500, oder Gemische davon.

Als Komponente (a) sind auch die Amide gleicher oder verschiedener ungesättigter Carbonsäuren von aromatischen, cycloaliphatischen und aliphatischen Polyaminen mit bevorzugt 2 bis 6, besonders 2 bis 4 Aminogruppen geeignet. Beispiele für solche Polyamine sind Ethylendiamin, 1,2- oder 1,3-Propylendiamin, 1,2-, 1,3- oder 1,4-Butylendiamin, 1,5-Pentylendiamin, 1,6-Hexylendiamin, Octylendiamin, Dodecylendiamin, 1,4-Diaminocyclohexan, Isophorondiamin, Phenylendiamin, Bisphenylendiamin, Di-β-aminoethylether, Diethylentriamin, Triethylentetramin, Di(β-aminoethoxy)- oder Di(β-aminopropoxy)ethan. Weitere geeignete Polyamine sind Polymere und Copolymere mit gegebenenfalls zusätzlichen Aminogruppen in der Seitenkette und Oligoamide mit Aminoendgruppen. Beispiele für solche ungesättigten Amide sind: Methylen-bis-acrylamid, 1,6-Hexamethylen-bis-acrylamid, Diethylentriamin-tris-methacrylamid, Bis(methacrylamidopropoxy)-ethan, β-Methacrylamidoethylmethacrylat, N[(β-Hydroxyethoxy)ethyl]-acrylamid.

Geeignete ungesättigte Polyester und Polyamide leiten sich z.B. von Maleinsäure und Diolen oder Diaminen ab. Die Maleinsäure kann teilweise durch andere Dicarbonsäuren ersetzt sein. Sie können zusammen mit ethylenisch ungesättigten Comonomeren, z.B. Styrol, eingesetzt werden. Die Polyester und Polyamide können sich auch von Dicarbonsäuren und ethylenisch ungesättigten Diolen oder Diaminen ableiten, besonders von längerkettigen mit z.B. 6 bis 20 C-Atomen. Beispiele für Polyurethane sind solche, die aus gesättigten oder ungesättigten Diisocyanaten und ungesättigten bzw. gesättigten Diolen aufgebaut sind.

Polybutadien und Polyisopren und Copolymere davon sind bekannt. Geeignete Comonomere sind z.B. Olefine wie Ethylen, Propen, Buten, Hexen, (Meth)-Acrylate, Acryl-nitril, Styrol oder Vinylchlorid. Polymere mit (Meth)-Acrylatgruppen in der Seitenkette sind ebenfalls bekannt. Es kann sich z.B. um Umsetzungsprodukte von Epoxidharzen auf Novolakbasis mit (Meth)-Acrylsäure handeln, um Homo- oder Copolymere des Vinylalkohols oder deren Hydroxyalkylderivaten, die mit (Meth)-Acrylsäure verestert sind, oder um Homo- und Copolymere von (Meth)-Acrylaten, die mit Hydroxyalkyl(meth)acrylaten verestert sind.

Die photopolymerisierbaren Verbindungen können alleine oder in beliebigen Mischungen eingesetzt werden. Bevorzugt werden Gemische von Polyol(Meth)Acrylaten verwendet.

Den erfindungsgemässen Zusammensetzungen können auch Bindemittel zugesetzt werden, was besonders zweckmässig ist, wenn es sich bei den photopolymerisierbaren Verbindungen um flüssige oder viskose Substanzen handelt. Die Menge des Bindemittels kann z.B. 5-95, vorzugsweise 10-90 und besonders 40-90 Gew.-% betragen, bezogen auf den Gesamtfestkörper. Die Wahl des Bindemittels erfolgt je nach dem Anwendungsgebiet und hierfür geforderter Eigenschaften wie Entwickelbarkeit in wässrigen und organischen Lösungsmittelsystemen, Adhäsion auf Substraten und Sauerstoffempfindlichkeit.

Geeignete Bindemittel sind z.B. Polymere mit einem Molekulargewicht von etwa 5000-2000000, bevorzugt 10000-1000000. Beispiele sind: Homo- und Copolymere Acrylate und Methacrylate, z.B. Copolymere aus Methylmethacrylat/Ethylacrylat/Methacrylsäure, Poly(methacrylsäurealkylester), Poly(acrylsäurealkylester); Celluloseester und -ether wie Celluloseacetat, Celluloseacetatbutyrat, Methylcellulose, Ethylcellulose; Polyvinylbutyral, Polyvinylformal, cyclisierter Kautschuk, Polyether wie Polyethylenoxid, Polypropylenoxid, Polytetrahydrofuran; Polystyrol, Polycarbonat, Polyurethan, chlorierte Polyolefine, Polyvinylchlorid, Copolymere aus Vinylchlorid/Vinylidenchlorid, Copolymere von Vinylidenchlorid mit Acrylnitril, Methylmethacrylat und Vinylacetat, Polyvinylacetat, Copoly(ethylen/vinylacetat), Polymere wie Polycaprolactam und Poly(hexamethylenadipamid), Polyester wie Poly(ethylenglykolterephtalat) und Poly(hexamethylenglykolsuccinat).

Die ungesättigten Verbindungen können auch im Gemisch mit nicht-photopolymerisierbaren filmbildenden Komponenten verwendet werden. Diese können z.B. physikalisch trocknende Polymere bzw. deren Lösungen in organischen Lösemitteln sein, wie z.B. Nitrocellulose oder Celluloseacetobutyrat. Diese können aber auch chemisch bzw. thermisch härtbare Harze sein, wie z.B. Polyisocyanate, Polyepoxide oder Melaminharze. Die Mitverwendung von thermisch härtbaren Harzen ist für die Verwendung in sogenannten Hybrid-Systemen von Bedeutung, die in einer ersten Stufe photopolymerisiert werden und in einer zweiten Stufe durch thermische Nachbehandlung vernetzt werden.

Die erfindungsgemässen Photoinitiatoren sind auch geeignet als Initiatoren für die Härtung von oxidativ trocknenden Systemen, wie sie z.B. im Lehrbuch der Lacke und Beschichtungen Band III, 296-328, Verlag W.A. Colomb in der Heenemann GmbH, Berlin-Oberschwandorf (1976) beschrieben sind.

Die photopolymerisierbaren Gemische können ausser dem Photoinitiator verschiedene Additive enthalten. Beispiele hierfür sind thermische Inhibitoren, die eine vorzeitige Polymerisation verhindern sollen, wie z.B. Hydrochinon, Hydrochinonderivate, p-Methoxyphenol, β-Naphthol oder sterisch gehinderte Phenole wie z.B. 2,6-Di(tert-butyl)-p-kresol. Zur Erhöhung der Dunkellagerstabilität können z.B. Kupferverbindungen, wie Kupfernaphthenat, -stearat oder -octoat, Phosphorverbindungen, wie z.B. Triphenylphosphin, Tributylphosphin, Triethylphosphit, Triphenylphosphit oder Tribenzylphosphit, quartäre Ammoniumverbindungen, wie z.B. Tetramethylammoniumchlorid oder Trimethyl-benzylammoniumchlorid, oder Hydroxylaminderivate, wie z.B. N-Diethylhydroxylamin verwendet werden. Zwecks Ausschluss des Luftsauerstoffes während der Polymerisation kann man Paraffin oder ähnliche wachsartige Stoffe zusetzen, die bei Beginn der Polymerisation wegen mangelnder Löslichkeit im Polymeren an die Oberfläche wandern und eine transparente Oberflächenschicht bilden, die den Zutritt von Luft verhindert. Ebenso kann eine sauerstoffundurchlässige Schicht aufgetragen werden. Als Lichtschutzmittel können UV-Absorber, wie z.B. solche vom Hydroxyphenyl-benztriazol-, Hydroxyphenyl-benzo-phenon-, Oxafsäureamid- oder Hydroxyphenyl-s-triazin-Typ, zugesetzt werden. Es können einzelne oder Mischungen dieser Verbindungen mit oder ohne Einsatz von sterisch ge-hinderten Aminen (HALS) verwendet werden.

Beispiele für solche UV-Absorber und Lichtschutzmittel sind
1. 2-(2'-Hydroxyphenyl)-benzotriazote, wie z.B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)-benzotriazol, 2-(2'-Hydroxy-5'-(1,1,3,3-tetramethylbutyl)phenyl)-benzotriazol, 2-(3',5'-Di-tert-butyl-2'-hydroxyphenyl)-5-chlor-benzotriazol, 2-(3'-tert-Butyl-2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'octoxyphenyl)-benzotriazol, 2-(3',5'-Di-tert-amyl-2'-hydroxyphenyl)-benzotriazol, 2-(3',5'-Bis-(α,α-dimethylbenzyl)-2'-hydroxyphenyl)-benzotriazol, Mischung aus 2-(3'-tert-Butyl-2'-hydroxy-5'-(2-octyloxycarbonylethyl)phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol, und 2-(3'-tert-Butyl-2'-hydroxy-5'-(2-isooctyloxycarbonylethyl)phenyl-benzotriazol, 2,2'-Methylen-bis[4-(1,1,3,3-tetramethylbutyl)-6-benzotriazol-2-yl-phenol]; Umesterungsprodukt von 2-[3'tert-Butyl-5'-(2-methoxycarbonylethyl)-2'-hydroxy-phenyl]-benzotriazol mit Polyethylenglycol 300; [R-CH₂CH₂-COO(CH₂)₃]₂- mit R = 3'-tert-Butyl-4'-hydroxy-5'-2H-benzotriazol-2-ylphenyl.
2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.
3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert- butyl-4-hydroxybenzoesäure-octadecylester, 3,5-Di-tert-butyl-4-hydroxybenzoesäure-2-methyl-4,6-di-tert-butylphenylester.
4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.
5. Sterisch gehinderte Amine, wie z.B. Bis-(2,2,6,6-tetramethyl-piperidyl)-sebacat, Bis-(2,2,6,6-tetramethyl-piperidyl)-succinat, Bis-(1,2,2,6,6-pentamethylpiperidyl)-sebacat, n-Butyl-3,5-di-tert-butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert-Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon), 4-Benzoyl-2,2,6,6-tetramethylpiperidin, 4-Stearyloxy-2,2,6,6-tetramethylpiperidin, Bis-(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro-[4.5]decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion.
6. Oxalsäurediamide, wie z.B. 4,4'-Di-octyloxy-oxanilid, 2,2'-Diethoxy-oxanilid, 2,2'-Di-octyloxy-5,5'-di-tert-butyl-oxanilid, 2,2'-Di-dodecyloxy-5,5'di-tert-butyl-oxanilid, 2-Ethoxy-2'-ethyl-oxanilid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.
7. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin, 2-[4-dodecyl/tridecyl-oxy-(2-hydroxypropyl)oxy-2-hydroxyphenyl]-4,6-bis(2,4-dimethylphenyl)-1,3,5-triazin.
8. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythrit-diphosphit, Bis-(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis-(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis-(2,4-di-tert-butylphenyl)-4,4'-biphenylen-diphosphonit, 6-lsooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz[d,g]-1,3,2-dioxaphosphocin, Bis-(2,4-di-tert-butyl-6-methylphenyl)-methylphosphit, Bis-(2,4-di-tert-butyl-6-methylphenyl)-ethylphosphit.

Erfindungsgemäss ist daher auch eine photopolymerisierbare Zusammensetzung, enthaltend als Photoinitiator mindestens eine Molekülkomplexverbindung aus einer Mono-, Bisoder Trisacylphosphinoxidverbindung mit einer α-Hydroxyketonverbindung, sowie einen UV-Absorber aus der Klasse der Hydroxyphenyl-s-triazine und/oder Hydroxyphenylbenztriazole und/oder sterisch gehinderten Amine auf Basis von 2,2,6,6-Tetramethyl-Piperidinen.

Zur Beschleunigung der Photopolymerisation können Amine zugesetzt werden, wie z.B. Triethanolamin, N-Methyl-diethanolamin, p-Dimethylaminobenzoesäure-ethylester oder Michlers Keton. Die Wirkung der Amine kann verstärkt werden durch den Zusatz von aromatischen Ketonen vom Typ des Benzophenons. Als Sauerstofffänger brauchbare Amine sind beispielsweise substituierte N,N-dialkylaniline, wie sie in der EP-A-339 841 beschrieben sind. Weitere Beschleuniger, Coinitiatoren und Autoxidizer sind Thiole, Thioether, Disulfide und Phosphine, wie z.B. in der EP-A-438 123 und GB-A-2 180 358 beschrieben.
Eine Beschleunigung der Photopolymerisation kann weiterhin durch Zusatz von Photosensibilisatoren geschehen, welche die spektrale Empfindlichkeit verschieben bzw. verbreitern. Dies sind insbesondere aromatische Carbonylverbindungen wie z.B. Benzophenon-, Thioxanthon-, Anthrachinon- und 3-Acylcumarinderivate sowie 3-(Aroylmethylen)-thiazoline, aber auch Eosin-, Rhodamin- und Erythrosin-Farbstoffe.

Unterstützt werden kann der Härtungsvorgang insbesondere von (z.B. mit Titandioxid) pigmentierten Zusammensetzungen, auch durch Zugabe einer unter thermischen Bedingungen radikalbildenden Komponente wie z.B. einer Azoverbindung wie etwa 2,2'-Azobis(4-methoxy-2,4-dimethylvaleronitril), eines Triazens, Diazosulfids, Pentazadiens oder einer Peroxyverbindung wie etwa Hydro-peroxid oder Peroxycarbonat, z.B. t-Butylhydroperoxid, wie z.B. in der EP-A 245 639 beschrieben.

Die erfindungsgemässen Zusammensetzungen können auch einen photoreduzierbaren Farbstoff, wie z.B. Xanthen-, Benzoxanthen, Benzothioxanthen, Thiazin-, Pyronin, Porphyrin- oder Acridinfarbstoffe, und/oder eine durch Strahlung spaltbare Trihalogenmethylverbindung enthalten. Ähnliche Zusammensetzungen sind beispielsweise in der EP-A-445 624 beschrieben.

Weitere übliche Zusätze sind - je nach Verwendungszweck - optische Aufheller, Füllstoffe, Pigmente, Farbstoffe, Netzmittel oder Verlaufhilfsmittel.
Zur Härtung dicker und pigmentierter Beschichtungen eignet sich der Zusatz von MikroGlaskugeln oder pulverisierter Glasfasern, wie z.B. im US-A-5,013,768 beschrieben.

Gegenstand der Erfindung sind auch Zusammensetzungen enthaltend als Komponente (a) mindestens eine in Wasser gelöste oder emulgierte ethylenisch ungesättigte photopolymerisierbare Verbindung.

Solche strahlungshärtbaren wässrigen Prepolymerdispersionen sind in vielen Variationen im Handel erhältlich. Man versteht darunter eine Dispersion aus Wasser und mindestens einem darin dispergierten Prepolymeren. Die Konzentration des Wassers in diesen Systemen liegt z.B. bei 5 bis 80, insbesondere 30 bis 60Gew.-%. Das strahlungshärtbare Prepolymere bzw. Prepolymerengemisch ist beispielsweise in Konzentrationen von 95 bis 20, insbesondere 70 bis 40Gew.-% enthalten. In diesen Zusammensetzungen ist die Summe der für Wasser und Prepolymere genannten Prozentzahlen jeweils 100, die Hilfs- und Zusatzstoffe kommen, je nach Verwendungszweck in unterschiedlichen Mengen hinzu.

Bei den strahlungshärtbaren, in Wasser dispergierten, oft auch gelösten filmbildenden Prepolymeren handelt es sich um für wässrige Prepolymerdispersionen an sich bekannte, durch freie Radikale initiierbare mono- oder polyfunktionelle ethylenisch ungesättigte Prepolymere, die beispielsweise einen Gehalt von 0,01 bis 1,0 Mol pro 100 g Prepolymer an polymerisierbaren Doppelbindungen, sowie ein mittleres Molekulargewicht von z.B. mindestens 400, insbesondere von 500 bis 10000 aufweisen. Je nach Anwendungszweck kommen jedoch auch Prepolymere mit höheren Molekulargewichten in Frage.
Es werden beispielsweise polymerisierbare C-C-Doppelbindungen enthaltende Polyester mit einer Säurezahl von höchstens 10, polymerisierbare C-C-Doppelbindungen enthaltende Polyether, hydroxylgruppenhaltige Umsetzungsprodukte aus einem mindestens zwei Epoxidgruppen pro Molekül enthaltenden Polyepoxid mit mindestens einer α,β-ethylenisch ungesättigten Carbonsäure, Polyurethan(meth-)acrylate sowie α,β-ethylenisch ungesättigte
Acrylreste enthaltende Acrylcopolymere verwendet, wie sie in der EP-A-12 339 beschrieben sind. Gemische dieser Prepolymeren können ebenfalls verwendet werden. In Frage kommen ausserdem die in der EP-A-33 896 beschriebenen polymerisierbaren Prepolymere, bei denen es sich um Thioetheraddukte von polymerisierbaren Prepolymeren mit einem mittleren Molekulargewicht von mindestens 600, einem Carboxylgruppengehalt von 0,2 bis 15 % und einem Gehalt von 0,01 bis 0,8 Mol polymerisierbarer C-C-Doppelbindungen pro 100 g Prepolymer handelt. Andere geeignete wässrige Dispersionen auf Basis von speziellen (Meth-)Acrylsäurealkylester-Polymerisaten sind in der EP-A-41 125 beschrieben, geeignete in Wasser dispergierbare, strahlungshärtbare Prepolymere aus Urethanacrylaten sind der DE-A-29 36 039 zu entnehmen.
Als weitere Zusätze können diese strahlungshärtbaren wässrigen Prepolymerdispersionen Dispergierhilfsmittel, Emulgatoren, Antioxidantien, Lichtstabilisatoren, Farbstoffe, Pigmente, Füllstoffe, z.B. Talkum, Gips, Kieselsäure, Rutil, Russ, Zinkoxid, Eisenoxide, Reaktionsbeschleuniger, Verlaufsmittel, Gleitmittel, Netzmittel, Verdickungsmittel, Mattierungsmittel, Entschäumer und andere in der Lacktechnologie übliche Hilfsstoffe enthalten. Als Dispergierhilfsmittel kommen wasserlösliche hochmolekulare organische Verbindungen mit polaren Gruppen, wie z.B. Polyvinylalkohole, Polyvinylpyrrolidon oder Celluloseether in Frage. Als Emulgatoren können nicht-ionische, gegebenenfalls auch ionische Emulgatoren verwendet werden.

Die photopolymerisierbaren Zusammensetzungen enthalten den Photoinitiator (b) zweckmässig in einer Menge von 0,05 bis 15 Gew.-%, vorzugsweise 0,1 bis 5 Gew.-%, bezogen auf die Zusammensetzung.

In bestimmten Fällen kann es von Vorteil sein, zusätzlich zur erfindungsgemässen Molekülkomplex-Photoinitiator-Verbindung weitere bekannte Photoinitiatoren zu verwenden, z.B. Benzophenon, Benzophenonderivate, Acetophenon, Acetophenonderivate, wie beispielsweise α-Hydroxycycloalkylphenylketon, Dialkoxyacetophenon, α-Hydroxy- oder α-Aminoacetophenon, 4-Aroyl-1,3-Dioxolan, Benzoinalkylether und Benzilketale, Monoacylphosphinoxide, Bisacylphosphinoxide, Ferrocene oder Titanocene.
Im Falle des Einsatzes der erfindungsgemäßen Molekülkomplex-Photoinitiatoren in Hybridsystemen werden zusätzlich zu den erfindungsgemäßen radikalischen Härtern kationische Photoinitiatoren wie z.B. Benzoylperoxid, aromatische Sulfonium-, Phosphonium- oder lodonium-Salze oder Cyclopentadienylaren-eisen(ll)-Komplexsalze verwendet.

Die photopolymerisierbaren Zusammensetzungen können für verschiedene Zwecke verwendet werden, beispielsweise als Druckfarbe, als Klarlack, als Weisslack, z.B. für Holz oder Metall, als Anstrichstoff, u.a. für Papier, Holz, Metall oder Kunststoff, als tageslichthärtbarer Anstrich für Bauten- und Straßenmarkierung, für photographische Reproduktionsverfahren, für holographische Aufzeichnungsmaterialien, für Bildaufzeichnungsverfahren oder zur Herstellung von Druckplatten, die mit organischen Lösemitteln oder wässrig-alkalisch entwickelbar sind, zur Herstellung von Masken für den Siebdruck, als Zahnfüllmassen, als Klebstoffe, als drucksensitive Klebstoffe, als Laminierharze, als Aetz- oder Permanentresists und als Lötstoppmasken für elektronische Schaltungen, zur Herstellung von dreidimensionalen Gegenständen durch Massenhärtung (UV-Härtung in transparenten Formen) oder nach dem Stereolithographie-Verfahren, wie es z.B. im US-A-4,575,330 beschrieben ist, zur Herstellung von Verbundwerkstoffen (z.B. styrolischen Polyestern, die gegebenenfalls Glasfasern und andere Hilfsstoffe, enthalten können) und anderen dickschichtigen Massen, zur Beschichtung oder Versiegelung von elektronischen Teilen oder als Ueberzüge für optische Fasern.
Die erfindungsgemäßen Molekülkomplexverbindungen können weiterhin als Initiatoren für Emulsionspolymerisationen, als Initiatoren einer Polymerisation für die Fixierung von Ordnungszuständen von flüssigkristallinen Mono- und Oligomeren, als Initiatoren für die Fixierung von Farbstoffen auf organischen Materialien eingesetzt werden.

In Lacken werden man häufig Gemische eines Prepolymeren mit mehrfach ungesättigten Monomeren verwendet, die ausserdem noch ein einfach ungesättigtes Monomer enthalten. Das Prepolymere ist hierbei in erster Linie für die Eigenschaften des Lackfilmes massgebend, durch seine Variation kann der Fachmann die Eigenschaften des gehärteten Filmes beeinflussen. Das mehrfach ungesättigte Monomere fungiert als Vernetzer, das den Lackfilm unlöslich macht. Das einfach ungesättigte Monomere fungiert als reaktiver Verdünner, mit dessen Hilfe die Viskosität herabgesetzt wird, ohne dass ein Lösungsmittel verwendet werden muss.

Ungesättigte Polyesterharze werden meist in Zweikomponentensystemen zusammen mit einem einfach ungesättigten Monomer, vorzugsweise mit Styrol, verwendet. Für Photoresists werden oft spezifische Einkomponentensysteme verwendet, wie z.B. Polymaleinimide, Polychalkone oder Polyimide, wie sie in der DE-A-23 08 830 beschrieben sind.

Die erfindungsgemässen Molekülkomplexverbindungen können weiterhin als radikalische Photoinitiatoren oder photoinitiierende Systeme für strahlenhärtbare Pulverlacke verwendet werden. Die Pulverlacke können auf festen Harzen und Monomeren enthaltend reaktive Doppelbindungen basieren, wie z.B. Maleaten, Vinylethern, Acrylaten, Acrylamiden und Mischungen davon. Ein radikalisch UV-härtbarer Pulverlack kann durch Mischen von ungesättigten Polyesterharzen mit festen Acrylamiden (z.B. Methylacrylamido-glycolatmethylester) und einem erfindungsgemässen radikalischen Photoinitiator, wie beispielsweise im Vortrag "Radiation Curing of Powder Coating", Conference Proceedings, Radtech Europe 1993 von M. Wittig und Th. Gohmann beschrieben, formuliert werden. Ebenso können radikalisch UV-härtbare Pulverlacke durch Mischen von ungesättigten Polyesterharzen mit festen Acrylaten, Methacrylaten oder Vinylethern und einem erfindungsgemässen Photoinitiator formuliert werden. Die Pulver-lacke können auch Bindemittel enthalten, wie sie z.B. in der DE-A-42 28 514 und der EP-A-636 669 beschrieben sind. Die UV-härtbaren Pulverlacke können auch weisse oder farbige Pigmente enthalten. So kann z.B. vorzugsweise Rutil-Titaniumdioxid bis zu Konzentrationen von 50 Gew.-% eingesetzt werden, um einen gehärteten Pulverlack mit gutem Abdeckungsvermögen zu erhalten. Das Verfahren beinhaltet normalerweise elektrostatisches oder tribostatisches Aufsprühen des Pulvers auf das Substrat, wie z.B. Metall oder Holz, Aufschmelzen des Pulvers durch Erwärmen und, nachdem ein glatter Film entstanden ist, Strahlenhärten des Überzugs mit ultraviolettem und/oder sichtbaren Licht, z.B. mit Quecksilbermitteldrucklampen, Metallhalogenidlampen oder Xenonlampen. Ein besonderer Vorteil der strahlenhärtbaren Pulverlacke im Vergleich zu den entsprechenden thermisch härtbaren liegt darin, dass die Fliesszeit nach dem Aufschmelzen der Pulverpartikel wahlweise hinausgezögert werden kann, um die Bildung eines glatten hochglänzenden Überzugs zu gewährleisten. Im Gegensatz zu thermisch härtbaren Systemen können strahlenhärtbare Pulverlacke ohne den unerwünschten Effekt der Lebensdauerverkürzung so formuliert werden, dass sie bei niedrigeren Temperaturen schmelzen. Aus diesem Grund sind sie auch geeignet als Überzüge für wärmeempfindliche Substrate, wie z.B. Holz oder Kunststoffe.
Die Pulverlackformulierungen können neben den erfindungsgemässen Molekülkomplex-Photoinitiatoren auch UV-Absorber enthalten. Entsprechende Beispiele sind vorstehend unter den Punkten 1.-8. aufgeführt.

Die erfindungsgemässen photohärtbaren Zusammensetzungen eignen sich z.B. als Beschichtungsstoffe für Substrate aller Art, z.B. Holz, Textilien, Papier, Keramik, Glas, Kunststoffe wie Polyester, Polyethylenterephthalat, Polyolefine oder Celluloseacetat, insbesondere in Form von Filmen, sowie Metalle wie Al, Cu, Ni, Fe, Zn, Mg oder Co und GaAs, Si oder SiO₂, auf denen eine Schutzschicht oder durch bildmässiges Belichten eine Abbildung aufgebracht werden soll.

Die Beschichtung der Substrate kann erfolgen, indem eine flüssige Zusammensetzung, eine Lösung oder Suspension auf das Substrat aufgebracht wird. Die Wahl des Lösungsmittels und die Konzentration richten sich hauptsächlich nach der Art der Zusammensetzung und nach dem Beschichtungsverfahren. Das Lösungsmittel soll inert sein, d.h. es soll mit den Komponenten keine chemische Reaktion eingehen und es soll bei der Trocknung nach dem Beschichten wieder entfernt werden können. Geeignete Lösungsmittel sind z.B. Ketone, Ether und Ester, wie Methylethylketon, Isobutylmethylketon, Cyclopentanon, Cyclohexanon, N-Methylpyrrolidon, Dioxan, Tetrahydrofuran, 2-Methoxyethanol, 2-Ethoxyethanol, 1-Methoxy-2-propanol, 1,2-Dimethoxyethan, Essigsäureethylester, Essigsäure-n-butylester und 3-Ethoxy-propionsäureethylester.
Die Formulierung wird mittels bekannter Beschichtungsverfahren gleichförmig auf ein Substrat aufgebracht, z.B. durch Schleudern, Tauchen, Rakelbeschichtung, Vorhanggiessverfahren, Aufpinseln, Sprühen, speziell durch elektrostatisches Sprühen und Reverse-Roll-Beschicht-ung. Es ist auch möglich, die lichtempfindliche Schicht auf einen temporären, flexiblen Träger zu bringen und dann durch Schichtübertragung via Lamination das endgültige Substrat, z.B. eine kupferkaschierte Leiterplatte, zu beschichten.

Die Auftragsmenge (Schichtdicke) und Art des Substrates (Schichtträger) sind abhängig vom gewünschten Applikationsgebiet. Der Schichtdickenbereich umfasst im allgemeinen Werte von ca 0,1 µm bis mehr als 10 µm.

Die erfindungsgemässen strahlungsempfindlichen Zusammensetzungen finden Anwendung als Negativresists, die eine sehr hohe Lichtempfindlichkeit aufweisen und schwellungsfrei in wässrig-alkalischem Medium entwickelt werden können. Sie eignen sich als Photoresists für die Elektronik (Galvanoresist, Aetzresist, Lötstopresist), die Herstellung von Druckplatten, wie Offsetdruckplatten oder Siebdruckformen, den Einsatz beim Formteilätzen oder den Einsatz als Mikroresist bei der Herstellung integrierter Schaltkreise. Dementsprechend unterschiedlich sind die möglichen Schichträger und die Verarbeitungsbedingungen der beschichteten Substrate.
Für photographische Informationsaufzeichnungen dienen z.B. Folien aus Polyester, Celluloseacetat oder mit Kunststoff beschichtete Papiere; für Offsetdruckformen speziell behandeltes Aluminium, für die Herstellung gedruckter Schaltungen kupferkaschierte Laminate und für die Herstellung von integrierten Schaltkreisen Siliziumwafer. Die Schichtdicken für photographische Materialien und Offsetdruckformen betragen in der Regel ca. 0,5 µm bis 10 µm, für gedruckte Schaltungen 0,4 µm bis ca. 2 µm.

Nach dem Beschichten der Substrate wird das Lösungsmittel in der Regel durch Trocknen entfernt, und es resultiert eine Schicht des Photoresists auf dem Träger.

Der Begriff "bildmässige" Belichtung beinhaltet sowohl die Belichtung durch eine Photomaske, die ein vorbestimmtes Muster enthält, beispielsweise ein Diapositiv, die Belichtung durch einen Laserstrahl, der beispielsweise computergesteuert über die Oberfläche des beschichteteten Substrates bewegt wird und auf diese Weise ein Bild erzeugt, sowie die Bestrahlung mit computergesteuerten Elektronenstrahlen.

Nach der bildmässigen Belichtung des Materials und vor der Entwicklung kann es vorteilhaft sein, für kürzere Zeit eine thermische Behandlung durchzuführen. Dabei werden nur die belichteten Teile thermisch gehärtet. Die angewandten Temperaturen liegen im allgemeinen bei 50-150 °C, bevorzugt bei 80-130 °C; die Zeit für die thermische Behandlung liegt in der Regel zwischen 0,25 und 10 Minuten.

Die photohärtbare Zusammensetzung kann weiterhin in einem Verfahren zur Herstellung von Druckformen oder Photoresists, wie es z.B. in der DE-A-40 13 358 beschrieben wird, verwendet werden. Darin wird die Zusammensetzung vor, zugleich mit oder nach der bildmäßigen Bestrahlung kurzzeitig mit sichtbarem Licht einer Wellenlänge von mindestens 400 nm ohne Maske belichtet.

Nach der Belichtung und gegebenenfalls thermischen Behandlung werden die unbelichteten Stellen des Photolacks in an sich bekannter Weise mit einem Entwickler entfernt.

Die erfindungsgemässen Zusammensetzungen sind -wie schon erwähnt- wässrig-alkalisch entwickelbar. Geeignete wässrig-alkalische Entwicklerlösungen sind insbesondere wässrige Lösungen von Tetraalkylammoniumhydroxiden oder von Alkalimetallsilikaten, -phosphaten, -hydroxiden und -carbonaten. Diesen Lösungen können gegebenenfalls noch kleinere Mengen an Netzmitteln und/oder organischen Lösungsmitteln zugesetzt sein. Typische organische Lösungsmittel, die den Entwicklerflüssigkeiten in kleinen Mengen zugesetzt werden können, sind beispielsweise Cyclohexanon, 2-Ethoxyethanol, Toluol, Aceton sowie Mischungen solcher Lösungsmittel.

Grosse Bedeutung hat die Photohärtung für Druckfarben, da die Trocknungszeit des Bindemittels ein massgeblicher Faktor für die Produktionsgeschwindigkeit graphischer Erzeugnisse ist und in der Grössenordnung von Bruchteilen von Sekunden liegen soll. Insbesondere für den Siebdruck sind UV-härtbare Farben von Bedeutung.

Gut geeignet sind die erfindungsgemässen Gemische -wie oben bereits erwähnt- auch zur Herstellung von Druckplatten. Hierbei werden z. B. Gemische von löslichen linearen Polyamiden oder Styrol/Butadien bzw. Styrol/Isopren Kautschuk, Polyacrylaten oder Polymethylmethacrylaten mit Carboxyl-Gruppen, Polyvinylalkoholen oder Urethanacrylaten mit photopolymerisierbaren Monomeren, beispielsweise Acryl- bzw. Methacrylamiden oder Acryl- bzw. Methacrylestern, und einem Photoinitiator verwendet. Filme und Platten aus diesen Systemen (nass oder trocken) werden über das Negativ (oder Positiv) der Druckvorlage belichtet und die ungehärteten Teile anschliessend mit einem geeigneten Lösemittel eluiert.

Ein weiteres Einsatzgebiet der Photohärtung ist die Metallbeschichtung, beispielsweise bei der Lackierung von Blechen und Tuben, Dosen oder Flaschenverschlüssen, sowie die Photohärtung auf Kunststoffbeschichtungen, beispielsweise von Fussboden- oder Wandbelägen auf PVC-Basis.

Beispiele für die Photohärtung von Papierbeschichtungen sind die farblose Lackierung von Etiketten, Schallplattenhüllen oder Buchumschlägen.

Ebenfalls interessant ist die Verwendung der erfindungsgemässen Verbindungen zur Härtung von Formteilen aus Verbundmassen. Die Verbundmasse besteht aus einem selbsttragenden Matrixmaterial, z.B. einem Glasfasergewebe, oder auch beispielsweise Pflanzenfasern [vgl. K.-P. Mieck, T. Reussmann in Kunststoffe 85 (1995), 366-370], das mit der lichthärtenden Formulierung durchtränkt wird. Mit den erfindungsgemässen Verbindungen hergestellte Formteile aus Verbundmassen erreichen eine hohe mechanische Stabilität und Widerstandsfähigkeit. Die erfindungsgemässen Verbindungen sind auch als Photohärter in Form-, Tränk- und Überzugsmassen, wie sie beispielsweise in der EP-A-7086 beschrieben sind, einsetzbar. Solche Massen sind beispielsweise Feinschichtharze, an die hohe Anforderungen bezüglich der Härtungsaktivität und Vergilbungsresistenz gestellt werden, faserverstärkte Formstoffe, wie z.B. plane, längs- oder quergewellte Lichtplatten. Verfahren zur Herstellung von solchen Formstoffen, wie z.B. Handauflegeverfahren, Faserspritz-, Schleuder- oder Wickelverfahren, sind z.B. von P.H. Selden in "Glasfaserverstärkte Kunststoffe", Seite 610, Springer Verlag Berlin-Heidelberg-New York 1967, beschrieben. Gebrauchsgegenstände, die beispielsweise nach diesem Verfahren hergestellt werden können sind Boote, beidseitig mit glasfaserverstärktem Kunststoff beschichtete Span- oder Tischlerplatten, Rohre, Behälter usw. Weitere Beispiele für Form-, Tränk- und Überzugsmassen sind UP-Harz-Feinschichten für glasfaserhaltige Formstoffe (GFK), z.B. Wellplatten und Papierlaminate. Papierlaminate können auf Harnstoff- oder Melaminharzen basieren. Die Feinschicht wird vor der Laminatherstellung auf einem Träger (z.B. einer Folie) erzeugt. Die erfindungsgemässen photohärtbaren Zusammensetzungen können auch für Giess-harze oder zur Einbettung von Gegenständen, z.B. von Elektronikteilen usw., verwendet werden. Zur Härtung werden Quecksilbermitteldrucklampen verwendet, wie sie in der UV-Härtung üblich sind. Von besonderem Interesse sind aber auch weniger intensive Lampen, z.B. vom Typ TL 40W/03 oder TL40W/05. Die Intensität dieser Lampen entspricht etwa dem Sonnenlicht. Es kann auch direktes Sonnenlicht zur Härtung eingesetzt werden. Ein weiterer Vorteil ist, dass die Verbundmasse in einem angehärteten, plastischen Zustand von der Lichtquelle entfernt und verformt werden kann. Danach erfolgt die vollständige Aushärtung.

Wichtig ist auch die Verwendung von photohärtbaren Zusammensetzungen für Abbildungsverfahren und zur optischen Herstellung von Informationsträgern. Hierbei wird -wie oben bereits beschrieben- die auf dem Träger aufgebrachte Schicht (nass oder trocken) durch eine Photomaske mit UV oder sichtbarem Licht bestrahlt und die unbelichteten Stellen der Schicht durch Behandlung mit einem Lösemittel (= Entwickler) entfernt. Das Aufbringen der photohärtbaren Schicht kann auch im Elektroabscheidungsverfahren auf Metall geschehen. Die belichteten Stellen sind vernetzt-polymer und dadurch unlöslich und bleiben auf dem Träger stehen. Bei entsprechender Anfärbung entstehen sichtbare Bilder. Ist der Träger eine metallisierte Schicht, so kann das Metall nach dem Belichten und Entwickeln an den unbelichteten Stellen weggeätzt oder durch Galvanisieren verstärkt werden. Auf diese Weise lassen sich gedruckte elektronische Schaltungen und Photoresists herstellen.

Die Lichtempfindlichkeit der erfindungsgemässen Zusammensetzungen reicht in der Regel vom UV-Gebiet (ca.200 nm) bis ca. 600 nm. Geeignete Strahlung enthält z.B. Sonnenlicht oder Licht, aus künstlichen Lichtquellen. Als Lichtquellen kommen daher eine grosse Anzahl der verschiedensten Typen zur Anwendung. Es sind sowohl Punktquellen als auch flächenförmige Strahler (Lampenteppiche) geeignet. Beispiele sind: Kohlelichtbogenlampen, Xenon-Lichtbogenlampen, Quecksilber-mitteldruck-, -hochdruck- und -niederdruckstrahler, gegebenenfalls mit Metall-Halogeniden dotiert (Metall-Halogenlampen), mikrowellenangeregte Metalldampflampen, Excimer Lampen, superaktinische Leuchtstoffröhren, Fluoreszenzlampen, Argonglühlampen, Blitzlampen, photographische Flutlichtlampen, Elektronenstrahlen und Röntgenstrahlen. Der Abstand zwischen Lampe und erfindungsgemässem zu belichtendem Substrat kann je nach Anwendungszweck und Lampentyp bzw. -stärke variieren, z.B. zwischen 2 cm und 150 cm. Geeignet sind z.B. auch Laser im sichtbaren Bereich.

Gegenstand der Erfindung ist daher auch ein Verfahren zur Photopolymerisation von Verbindungen mit ethylenisch ungesättigten Doppelbindungen, dadurch gekennzeichnet, dass eine wie oben beschriebene erfindungsgemässe Zusammensetzung mit Licht im Bereich von 200 bis 600 nm bestrahlt wird.

Erfindungsgegenstand ist auch die Verwendung der oben beschriebenen Zusammensetzung zur Herstellung von Lacken, Druckfarben, Druckplatten, Dentalmassen, Resistmaterialien sowie als Bildaufzeichnungsmaterial, insbesondere für holographische Aufzeichnungen.

Ebenfalls Gegenstand der Erfindung ist ein beschichtetes Substrat, das auf mindestens einer Oberfläche mit einer wie oben beschriebenen Zusammensetzung beschichtet ist, sowie ein Verfahren zur photographischen Herstellung von Reliefabbildungen, in welchem ein beschichtetes Substrat bildmässig belichtet wird und danach die unbelichteten Anteile mit einem Lösemittel entfernt werden. Die Belichtung kann dabei sowohl durch eine Maske als auch mittels eines Laserstrahls ohne Maske erfolgen.

Die erfindungsgemässen Molekülkomplexverbindungen lassen sich gut in die zu härtenden Formulierungen einarbeiten.
Die erfindungsgemässen Molekülkomplexverbindungen enthalten in der Regel weniger Verunreinigungen als die einzelnen Komponenten, da bei der Herstellung die Verunreinigungen in Lösung bleiben. Diese Photoinitiatoren sind daher auch für sehr empfindliche Anwendungen geeignet.
Die erfindungsgemässen Molekülkomplexverbindungen weisen eine gute Lagerstabilität auf.
Bei der Herstellung der Molekülkomplexverbindungen können auch Gemische zwischen den erfindungsgemässen Molekülkomplexkristallen und einer der zu deren Herstellung verwendeten Komponenten entstehen, z.B. eine Mischung aus Molekülkomplexkristallen bestehend aus Verbindungen der Formel I und II und Kristallen der Verbindungen der Formel I.
Auch diese Gemische sind als Photoinitiatoren einsetzbar.

Die nachfolgenden Beispiele erläutern die Erfindung weiter. Angaben in Teilen oder Prozenten beziehen sich, ebenso wie in der übrigen Beschreibung und in den Patent-ansprüchen, auf das Gewicht, sofern nichts anderes angegeben ist.

### Beispiel 1: Molekülkomplex aus Bis(2,6-dimethoxybenzoyl)-2,4,4-trimethylpentylphosphinoxid und α-Hydroxycyclohexylphenylketon

Zur Herstellung von Impfkristallen werden Bis(2,6-dimethoxybenzoyl)-2,4,4-trimethylpentylphosphinoxid und α-Hydroxycyclohexylphenylketon im Molverhältnis 1:1 in einer Mischung aus Isooctan und Ethylacetat (Gewichtsverhältnis 2.3:1) bei 80°C gelöst. Bei 53-55°C wird durch Reiben an der Glaswand mittels eines Glasstabes die Keimbildung initiiert, bis der Kristallisationsprozess eintritt. Die so erhaltenen Kristalle werden bei der erstmaligen Herstellung grösserer Mengen des Molekülkomplexes als Impfkristalle verwendet.
Herstellung grösserer Mengen der Molekülkomplexkristalle:
385 g Bis(2,6-dimethoxybenzoyl)-2,4,4-trimethylpentyl-phosphinoxid und 165 g α-Hydroxycyclohexylphenylketon werden in einem Gemisch aus 385 g Isooctan und 165 g Ethylacetat bei 80°C gelöst. Die Mischung wird auf 53-55°C abgekühlt. Bei dieser Temperatur wird die vorliegende Emulsion mit der entsprechenden Mischkristallmodifikation angeimpft und kristallisiert. Nach der Filtration bei 20°C wird das Produkt mit dem Lösungsmittel-Gemisch gewaschen und bei ca. 70°C und 50 mbar getrocknet. Es werden 530 g kristallines Trockenprodukt, d.h. 96% d.Theorie, erhalten. Der Schmelzpunkt (bestimmt durch Differential Scanning Calorimetry) beträgt 90°C. Der Phosphorgehalt ist 4.47%. Der Gehalt an Bis(2,6-dimethoxybenzoyl)-2,4,4-trimethylpentyl-phosphinoxid beträgt 71%, der an α-Hydroxycyclohexylphenylketon 29%. Diese Werte werden durch High Pressure Liquid Chromatography (HPLC) bestimmt.
Die Röntgenstrukturanalyse (gemessen an einem plättchenförmigen gelben Kristall mit einem Vierkreisdiffraktometer Philips PW1100, MoKα₁-Strahlung (λ = 0.70926 Å), berechnet mit direkten Methoden mit dem Programmsystem SHELX86 (Sheldrick, Göttingen) ergibt ein monoklines Kristallsystem mit der Raumgruppe P2₁/c (International Tables for X-ray Crystallography, 1974, Vol.IV).
Formel C₂₆H₃₅O₇P • C₁₃H₁₆O₂ ; Molekulargewicht 694.80
Die Elementarzelle enthält 4 Moleküle, die Abmessungen der Elementarzelle betragen:
a(Å) 17.514(2); b(Å) 10.518(1); c(Å) 20.912(2);
β(°) 97.92(1); V (Å³) 3815.5(8).

Die Verfeinerungsrechnung ergibt einen R-Wert von 0.047.

### Beispiel 2: Molekülkomplex aus Bis(2,6-dimethoxybenzoyl)-2,4,4-trimethylpentylphosphinoxid und α-Hydroxycyclohexylphenylketon

Die Herstellung der Impfkristalle erfolgt wie im Beispiel 1 beschrieben.
140 g Bis(2,6-dimethoxybenzoyl)-2,4,4-trimethylpentyl-phosphinoxid und 60 g α-Hydroxycyclohexylphenylketon werden in einem Gemisch aus 92 g Methylethylketon (MEK) und 11 g Wasser bei 30-35°C gelöst. (Die Anwesenheit von Wasser ist hier nicht zwingend. Der Komplex wird auch bei der Verwendung von reinem MEK erhalten) Die Lösung wird auf 20-23°C gekühlt und mit der entsprechenden Mischkristallmodifikation angeimpft. Sobald eine deutliche Kristallsuspension gebildet ist, wird langsam mit 500 ml Wasser verdünnt. Die Filtration bei 20°C ergibt 220 g Feuchtprodukt, welches mit Wasser gewaschen und bei ca. 70°C und 50 mbar getrocknet wird. Es werden 200 g, d.h. >99% d.Theorie, kristallines Trockenprodukt mit einem Schmelzpunkt von 91°C (DSC) erhalten. Der Gehalt an Bis(2,6-dimethoxybenzoyl)-2,4,4-trimethylpentyl-phosphinoxid beträgt 71%, der an α-Hydroxycyclohexylphenylketon 29% (HPLC).

### Beispiel 3: Molekülkomplex aus Bis(2,6-dimethoxybenzoyl)-2,4,4-trimethylpentylphosphinoxid und 2-Hydroxy-2-methyl-1-phenyl-propan-1-on

Die Herstellung von Impfkristallen erfolgt durch Lösen der Komponenten im Molverhältnis 1:1 bei Raumtemperatur in Essigester. Die Kristallisation wird durch Zugabe von Hexan erzwungen. Es bildet sich ein Gemisch aus Molekülkomplexkristallen bestehend aus Bis-(2,6-dimethoxybenzoyl)-2,4,4-trimethylpentyl-phosphinoxid und 2-Hydroxy-2-methyl-1-phenyl-propan-1-on und Kristallen bestehend aus Bis(2,6-dimethoxybenzoyl)-2,4,4-tri-methylpentylphosphinoxid. Dieses Kristallgemisch eignet sich bei der Herstellung grösserer Mengen von Kristallen des Molekülkomplexes als Impfkristall.
Herstellung grösserer Mengen der Molekülkomplexkristalle:
150 g Bis(2,6-dimethoxybenzoyl)-2,4,4-trimethylpentyl-phosphinoxid und 50 g 2-Hydroxy-2-methyl-1-phenyl-propan-1-on werden in einem Gemisch aus 92 g Methylethylketon und 11 g Wasser bei 30-35°C gelöst. (Die Anwesenheit von Wasser ist hier nicht zwingend. Der Komplex wird auch bei der Verwendung von reinem MEK erhalten). Die Lösung wird auf 5°C abgekühlt und mit der entsprechenden Mischkristallmodifikation angeimpft. Sobald bei 5°C eine deutliche Kristallsuspension gebildet ist, wird langsam mit 1000 ml Wasser verdünnt. Die Filtration bei 20°C und Trocknung bei ca. 60°C und 50 mbar ergibt 194 g kristallines Trockenprodukt, d.h. 97% d.Theorie, mit einem Schmelzpunkt von 73°C (DSC) und einem Phosphorgehalt von 4.7%.
Die Röntgenstrukturanalyse (gemessen an einem plättchenförmigen farblosen Kristall mit einem Vierkreisdiffraktometer Philips PW1100, MoKα₁-Strahlung (λ = 0.70926 Å), berechnet mit direkten Methoden mit dem Programmsystem SHELX86 (Sheldrick, Göttingen) ergibt ein monoklines Kristallsystem mit der Raumgruppe P2₁/n (International Tables for X-ray Crystallography, 1974, Vol.IV).
Formel C₂₆H₃₅O₇P • C₁₀H₁₂O₂; Molekulargewicht 654.73.
Die Elementarzelle enthält 4 Moleküle, die Abmessungen der Elementarzelle betragen:
a(Å) 17.828(2); b(Å) 10.365(1); c(Å) 19.592(2);
β (°) 95.46(1); V (Å³) 3603.9(8).

Die Verfeinerungsrechnung ergibt einen R-Wert von 0.042.

### Beispiel 4: Molekülkomplex aus Bis(phenyl)-2,4,6-trimethylbenzoyl-phosphinoxid und α-Hydroxycyclohexylphenylketon

Impfkristalle werden durch sehr langsames Abkühlen einer Lösung von Bis(phenyl)-2,4,6-trimethylbenzoyl-phosphinoxid und α-Hydroxycyclohexylphenylketon im Mol-Verhältnis 1:1 in ®Isopar E (Gemisch aus unverzweigten und verzweigten paraffinischen Kohlenwasserstoffen; ESSO) hergestellt.
Herstellung grösserer Mengen der Molekülkomplexkristalle
100 g Bis(phenyl)-2,4,6-trimethylbenzoyl-phosphinoxid und 59 g α-Hydroxycyclohexylphenylketon werden bei 80 °C in 200 ml ®Isopar E gelöst. Die erhaltene Lösung wird auf 55°C abgekühlt, wobei eine trübe Emulsion entsteht. Diese wird bei 53-55°C mit der entsprechenden Mischkristallmodifikation geimpft. Das Produkt kristallisiert dabei in blassgelben harten Kristallen aus. Anschliessend wird innerhalb von 2 bis 3 Stunden gleichmässig auf Raumtemperatur abgekühlt, und die erhaltene Suspension filtriert. Der Filterkuchen wird zunächst mit ®Isopar E, danach mit Hexan gewaschen. Die Trocknung erfolgt bei 50°C und 50 mbar. Es werden 150 g des Produkts, entsprechend 94.3% d.Theorie, erhalten. Der Schmelzpunkt liegt bei 69.4°C (DSC), der Phosphorgehalt beträgt 5.5%.
Die Röntgenstrukturanalyse (gemessen mit einem Vierkreisdiffraktometer Philips PW1100, MoKα₁-Strahlung (λ = 0.70926 Å), berechnet mit direkten Methoden mit dem Programmsystem SHELX86 (Sheldrick, Göttingen) ergibt ein triklines Kristallsystem mit der Raumgruppe Pl, zentrosymmetrisch (Nr 2.der "International Tables for X-ray Crystallography, 1974, Vol.IV").
Formel: C₂₂H₂₁O₂P · C₁₃H₁₆O₂; Molekulargewicht 552.65.
Die Elementarzelle enthält 2 Moleküle, die Abmessungen der Elementarzelle betragen:
a(Å) 9.081; b(Å) 11.436; c(Å) 16.092;
α(°) 91.98; β(°) 101.05; γ (°) 109.50; V(Å³) 1537.3.

Die Verfeinerungsrechnung ergibt einen R-Wert von 0.051.
Der Abstand der beiden über eine Wasserstoffbrückenbindung assoziierten O-Atome (OH-Gruppe des Hydroxyketons mit O-Atom am Phosphor des Phosphinoxids) beträgt 2.715 Å. Der Bindungswinkel O-H··O wird mit 169° gemessen.

### Beispiel 5: Molekülkomplex aus Bis(phenyl)-2,4,6-trimethylbenzoyl-phosphinoxid und α-Hydroxycyclohexylphenylketon (Schmelzverfahren)

Es werden 10 g Bis(phenyl)-2,4,6-trimethylbenzoyl-phosphinoxid und 5.9 g α-Hydroxycyclohexylphenylketon bei 100°C aufgeschmolzen, homogenisiert und langsam auf Raumtemperatur abkühlen lassen. Beim Animpfen mit der entsprechenden Molekülkomplexverbindung erfolgt die spontane Kristallisation.

### Beispiel 6: Molekülkomplex aus Bis(2,4,6-trimethylbenzoyl)-2,4-di-hexoxyphenyl-phosphin oxid und α-Hydroxycyclohexylphenylketon (Schmelzverfahren)

Impfkristalle werden durch sehr langsames Abkühlen einer 1:1 Schmelze aus Bis(2,4,6-trimethylbenzoyl)-2,4-di-hexoxyphenyl-phosphinoxid und α-Hydroxycyclohexylphenylketon und intensivem Kratzen an der Kolbenwand nach dem Erkalten der Schmelze erhalten. Herstellung durch Kristallisation
1.5 g Bis(2,4,6-trimethylbenzoyl)-2,4-di-hexoxyphenyl-phosphinoxid und 0.5 g α-Hydroxycyclohexylphenylketon werden bei 50-60°C in 10 ml ®Isopar E/Essigester (3:1) gelöst. Die erhaltene Lösung wird auf 20-25°C abgekühlt und mit Impfkristallen -wie oben beschrieben erhalten- beimpft. Anschliessend wird die Lösung über Nacht im Eisschrank aufbewahrt. Das Produkt kristallisiert dabei in blassgelben Kristallen aus. Die erhaltene Suspension wird filtriert und der Filterkuchen zunächst mit kaltem Isopar®E, danach mit kaltem Hexan gewaschen. Die Trocknung erfolgt bei 40-50°C und 50 mbar. Es wird 1 g des Produktes (ca. 50% d.Theorie) erhalten. Der Schmelzpunkt, bestimmt mit der DSC-Methode beträgt 67,3°C.

Die Röntgenstrukturanalyse (gemessen an einem würfelförmigen Kristall mit einem Vierkreisdiffraktometer Nonius CAD4 (Enraf Nonius), CuKα₁-Strahlung (λ = 1.54178 Å), berechnet mit direkten Methoden mit dem Programmsystem SHELX86, Sheldrick, Göttingen) ergibt ein triklines Kristallsystem mit der Raumgruppe P-1 (International Tables for X-ray Crystallography, 1974, Vol.IV).
Formel: C₃₈H₅₁O₅P • C₁₃H₁₆O₂; Molekulargewicht 823.02.
Die Elementarzelle enthält 2 Moleküle, die Abmessungen der Elementarzelle betragen:
a(Å) 11.721(1); b(Å) 12.327(1); c(Å) 17.493(1);
α(°) 105.73(1); β(°) 99.32(1); γ(°) 92.71(1); V (Å³) 2389.6(3).

Die Verfeinerungsrechnung ergibt einen R-Wert von 0.079.
Der Abstand der beiden über eine Wasserstoffbrückenbindung assoziierten O-Atome (OH-Gruppe des Hydroxyketons mit O-Atom am Phosphor des Phosphinoxids) beträgt 2.747 Å. Der Bindungswinkel O-H··O wird mit 169° gemessen.

### Beispiel 7: Härtung eines Weisslacks

Es wird ein photohärtbarer Weisslack hergestellt durch Mischen der folgenden Komponenten:

| | |
|---|---|
| 67.5 % | Ebecryl® 830 |
| 5.0 % | Hexandioldiacrylat |
| 2.5 % | Trimethylolpropantriacrylat |
| 25.0 % | RTC-2® Titandioxid und |
| 3.0 % | der Molekülkomplexverbindung aus Beispiel 3. |

Die Formulierung wird mit einer 100 µm Spaltrakel auf Spanplatten aufgetragen. Dann erfolgt die Belichtung mit einer 80 W/cm Quecksilbermitteldrucklampe des Typs Canrad-Hanovia (USA), wobei die Probe mit einer Geschwindigkeit von 5 m/min auf einem Band unter der Lampe hindurchgeführt wird. Es wird eine wischfeste und durchgehärtete Lackschicht erhalten, deren Pendelhärte (nach König, DIN 53157) 146 Sekunden beträgt.

## Patentansprüche

1. Molekülkomplexverbindung aus einer Mono-, Bis- oder Trisacylphosphinoxid-Verbindung mit einer α-Hydroxyketon-Verbindung.

2. Molekülkomplexverbindung nach Anspruch 1, worin die Mono-, Bis- oder Trisacylphosphinoxid-Verbindung eine Verbindung der Formel I 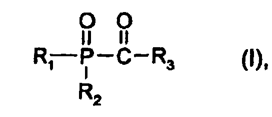 worin
**R**_{**1**} und **R**_{**2**} unabhängig voneinander C₁-C₁₂-Alkyl, Benzyl, unsubstituiertes oder ein- bis vierfach mit Halogen, C₁-C₈-Alkyl und/oder C₁-C₈-Alkoxy substituiertes Phenyl, Cyclohexyl oder eine Gruppe COR₃ darstellen; oder
**R**_{**1**} -OR₄, oder eine Gruppe 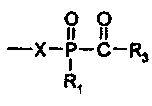 ist;
**R**_{**3**} unsubstituiertes oder ein- bis vierfach mit C₁-C₈-Alkyl, C₁-C₈-Alkoxy, C₁-C₈-Alkylthio und/oder Halogen substituiertes Phenyl oder eine Gruppe 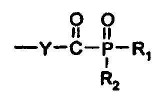 darstellt;
**R**_{**4**} C₁-C₈-Alkyl, Phenyl oder Benzyl bedeutet;
**Y** Phenylen, C₁-C₁₂-Alkylen oder Cyclohexylen ist;
**X** C₁-C₁₈-Alkylen oder eine Gruppe 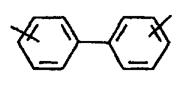 bedeutet, ist.

3. Molekülkomplexverbindung nach Anspruch 1, worin die α-Hydroxyketon-Verbindung eine Verbindung der Formel II 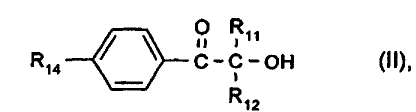 worin
**R**_{**11**} und **R**_{**12**} unabhängig voneinander Wasserstoff, C₁-C₆-Alkyl oder Phenyl bedeuten, oder
**R**_{**11**} und **R**_{**12**} zusammen mit dem Kohlenstoff-Atom, an welches sie gebunden sind, einen Cyclohexylring bilden;
**R**_{**14**} Wasserstoff, C₁-C₁₂-Alkyl, C₁-C₁₂-Alkoxy, -OCH₂CH₂-OR₁₅, eine Gruppe CH₂=C(CH₃)- oder 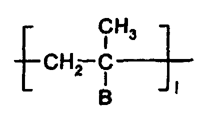 darstellt;
**I** für eine Zahl von 2 bis 10 steht;
**B** den Rest 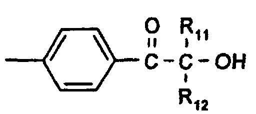 darstellt; und
**R**_{**15**} Wasserstoff, 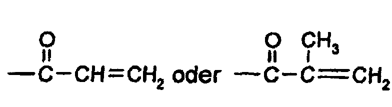 ist.

4. Molekülkomplexverbindung nach Anspruch 1, aus einer Mono-, Bis- oder Trisacylphosphinoxid-Verbindung der Formel I 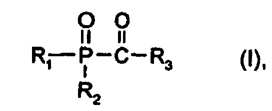 worin
**R**_{**1**} und **R**_{**2**} unabhängig voneinander C₁-C₁₂-Alkyl, unsubstituiertes oder ein- oder zweifach mit C₁-C₈-Alkyl, C₁-C₈-Alkoxy substituiertes Phenyl oder COR₃ bedeuten;
**R**_{**3**} für einen Rest 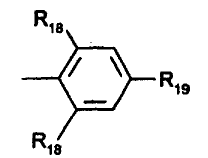 steht;
**R**_{**18**} C₁-C₄-Alkyl oder C₁-C₄-Alkoxy ist; und
**R**_{**19**} für Wasserstoff oder C₁-C₄-Alkyl steht;
und einer α-Hydroxyketonverbindung der Formel II 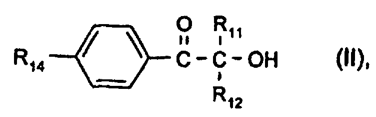 worin
**R**_{**11**} und **R**_{**12**} unabhängig voneinander C₁-C₄-Alkyl bedeuten oder R₁₁ und R₁₂ zusammen mit dem Kohlenstoffatom, an welches sie gebunden sind, einen Cyclohexylring bilden; und
**R**_{**14**} für Wasserstoff steht.

5. Molekülkomplexverbindung nach Anspruch 1, worin die Acylphosphinoxid-Verbindung Bis(2,6-dimethoxybenzoyl)-(2,4,4-trimethyl-pentyl)-phosphinoxid oder 2,4,6-Trimethylbenzoyl-diphenylphosphinoxid ist und die α-Hydroxyketon-Verbindung eine Verbindung der Formel II ist, worin R₁₁ und R₁₂ für C₁-C₄-Alkyl stehen oder R₁₁ und R₁₂ zusammen mit dem C-Atom, an welches sie gebunden sind, einen Cyclohexylring bilden, und R₁₄ Wasserstoff ist.

6. Molekülkomplexverbindung nach Anspruch 1, worin die Acylphosphinoxid-Verbindung Bis(2,6-dimethoxybenzoyl)-(2,4,4-trimethyl-pentyl)-phosphinoxid, Bis(2,4,6-trimethylbenzoyl)-(2,4-dihexyloxyphenyl)-phosphinoxid, Bis(2,4,6-trimethylbenzoyl)-(4-ethoxyphenyl)-phosphinoxid oder 2,4,6-Trimethylbenzoyl-diphenylphosphinoxid ist und die α-Hydroxyketon-Verbindung α-Hydroxy-cyclohexyl-phenyl-keton oder 2-Hydroxy-2-methyl-1-phenyl-propan-1-on ist.

7. Molekülkomplexverbindung nach Anspruch 1, enthaltend die Mono-, Bis- oder Trisacylphosphinoxid-Verbiridung und die α-Hydroxyketon-Verbindung im Molverhältnis 1:1.

8. Photopolymerisierbare Zusammensetzung, enthaltend
(a) mindestens eine ethylenisch ungesättigte photopolymerisierbare Verbindung und
(b) als Photoinitiator mindestens eine Molekülkomplexverbindung gemäss Anspruch 1.

9. Photopolymerisierbare Zusammensetzung gemäss Anspruch 8, die neben der Komponente (b) noch andere Additive enthält.

10. Photopolymerisierbare Zusammensetzung nach Anspruch 8, enthaltend 0.015-15, insbesondere 0.2-5 Gew.-% der Komponente (b) als Photoinitiator.

11. Photopolymerisierbare Zusammensetzung nach Anspruch 8, enthaltend als Photoinitiator mindestens eine Molekülkomplexverbindung gemäss Anspruch 1, sowie einen UV-Absorber aus der Klasse der Hydroxyphenyl-s-triazine und/oder Hydroxyphenylbenztriazole und/oder sterisch gehinderten Amine auf Basis von 2,2,6,6-Tetramethyl-Piperidinen.

12. Verfahren zur Photopolymerisation von Verbindungen mit ethylenisch ungesättigten Doppelbindungen, **dadurch gekennzeichnet, dass** eine Zusammensetzung nach Anspruch 8 mit Licht im Bereich von 200 bis 600 nm bestrahlt wird.

13. Verwendung der Zusammensetzung nach Anspruch 8 zur Herstellung von Lacken, Druckfarben, Druckplatten, Dentalmassen, Resistmaterialien sowie als Bildaufzeichnungsmaterial, insbesondere für holographische Aufzeichnungen.

14. Beschichtetes Substrat, das auf mindestens einer Oberfläche mit einer Zusammensetzung nach Anspruch 8 beschichtet ist.

15. Verfahren zur photographischen Herstellung von Reliefabbildungen, in welchem ein beschichtetes Substrat nach Anspruch 14 bildmässig belichtet wird und danach die unbelichteten Anteile mit einem Lösemittel entfernt werden.

## Claims

1. A molecular complex compound comprising a mono-, bis- or trisacylphosphine oxide compound with an α-hydroxy ketone compound.

2. A molecular complex compound according to claim 1, wherein the mono-, bis- or trisacylphosphine oxide compound is a compound of the formula I 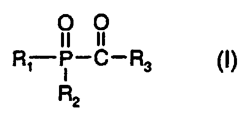 in which
**R**_{**1**} and **R**_{**2**} independently of one another are C₁-C₁₂alkyl, benzyl, phenyl which is unsubstituted or substituted from one to four times by halogen, C₁-C₈alkyl and/or C₁-C₈alkoxy, or are cyclohexyl or a group COR₃; or
**R**_{**1**} is -OR₄, or a group 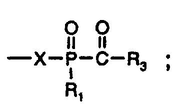 **R**_{**3**} is phenyl which is unsubstituted or substituted from one to four times by C₁-C₈alkyl, C₁-C₈alkoxy, C₁-C₈alkylthio and/or halogen, or is a group 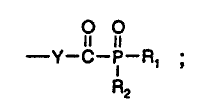 **R**_{**4**} is C₁-C₈alkyl, phenyl or benzyl;
**Y** is phenylene, C₁-C₁₂alkylene or cyclohexylene; and
**X** is C₁-C₁₈alkylene or a group

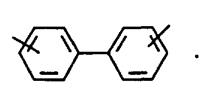

3. A molecular complex compound according to claim 1, wherein the α-hydroxy ketone compound is a compound of the formula II 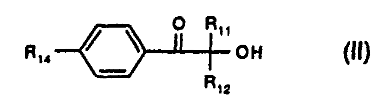 in which
**R**_{**11**} and **R**_{**12**} independently of one another are hydrogen, C₁-C₆alkyl or phenyl, or **R**_{**11**} and **R**_{**12**}, together with the carbon atom to which they are attached, form a cyclohexyl ring;
**R**_{**14**} is hydrogen, C₁-C₁₂alkyl, C₁-C₁₂alkoxy, -OCH₂CH₂-OR₁₅, a group CH₂=C(CH₃)- or 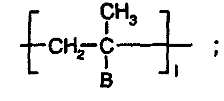 **I** is a number from 2 to 10;
**B** is the radical 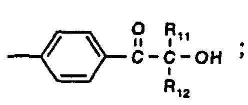 and
**R**_{**15**} is hydrogen,

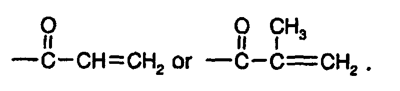

4. A molecular complex compound according to claim 1, comprising a mono-, bis- or trisacylphosphine oxide compound of the formula I 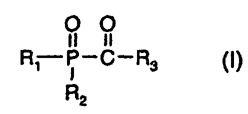 in which
**R**_{**1**} and **R**_{**2**} independently of one another are C₁-C₁₂alkyl, phenyl which is unsubstituted or substituted once or twice by C₁-C₆alkyl and/or C₁-C₈alkoxy, or are COR₃;
**R**_{**3**} is a radical 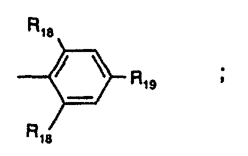 **R**_{**18**} is C₁-C₄alkyl or C₁-C₄alkoxy; and
**R**_{**19**} is hydrogen or C₁-C₄alkyl;
and an α-hydroxy ketone compound of the formula II 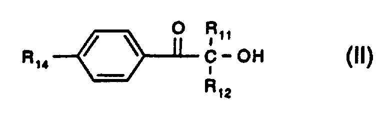 in which
**R**_{**11**} and **R**_{**12**} independently of one another are C₁-C₄alkyl or R₁₁ and R₁₂, together with the carbon atom to which they are attached, form a cyclohexyl ring; and
**R**_{**14**} is hydrogen.

5. A molecular complex compound according to claim 1, wherein the acylphosphine oxide compound is bis(2,6-dimethoxybenzoyl)(2,4,4-trimethylpentyl)phosphine oxide or 2,4,6-trimethylbenzoyldiphenylphosphine oxide and the α-hydroxy ketone compound is a compound of the formula II in which R₁₁ and R₁₂ are C₁-C₄alkyl or R₁₁ and R₁₂, together with the C atom to which they are attached, form a cyclohexyl ring, and R₁₄ is hydrogen.

6. A molecular complex compound according to claim 1, wherein the acylphosphine oxide compound is bis(2,6-dimethoxybenzoyl)(2,4,4-trimethylpentyl)phosphine oxide, bis(2,4,6-trimethylbenzoyl)(2,4-dihexyloxyphenyl)phosphine oxide, bis(2,4,6-trimethyl-benzoyl)(4-ethoxyphenyl)phosphine oxide or 2,4,6-trimethylbenzoyldiphenylphosphine oxide and the α-hydroxy ketone compound is α-hydroxycyclohexyl phenyl ketone or 2-hydroxy-2-methyl-1-phenylpropan-1-one.

7. A molecular complex compound according to claim 1, comprising the mono-, bis- or trisacylphosphine oxide compound and the α-hydroxy ketone compound in a molar ratio of 1:1.

8. A photopolymerizable composition comprising
(a) at least one ethylenically unsaturated photopolymerizable compound, and
(b) as photoinitiator, at least one molecular complex compound according to claim 1.

9. A photopolymerizable composition according to claim 8, which comprises other additives in addition to component (b).

10. A photopolymerizable composition according to claim 8, containing 0.015-15, in particular 0.2-5 % by weight of component (b) as photoinitiator.

11. A photopolymerizable composition according to claim 8, comprising as photoinitiator at least one molecular complex compound according to claim 1 and also a UV absorber from the class of the hydroxyphenyl-s-triazines and/or hydroxyphenylbenzo-triazoles and/or sterically hindered amines based on 2,2,6,6-tetramethylpiperidines.

12. A method of photopolymerizing compounds having ethylenically unsaturated double bonds, which comprises irradiating a composition according to claim 8 with light in the range from 200 to 600 nm.

13. The use of a composition according to claim 8 for producing paints, printing inks, printing plates, dental compositions and resist materials and as image recording material, especially for holographic recordings.

14. A coated substrate which is coated on at least one surface with a composition according to claim 8.

15. A process for the photographic production of relief images, in which a coated substrate according to claim 14 is subjected to imagewise exposure and then the unexposed portions are removed with a solvent.

## Revendications

1. Combinaison complexe moléculaire à partir d'un composé d'oxyde de mono-, bis- ou trisacylphosphine et d'un composé d'α-hydroxycétone.

2. Combinaison complexe moléculaire selon la revendication 1, où le composé d'oxyde de mono-, bis- ou trisacylphosphine représente un composé de formule I 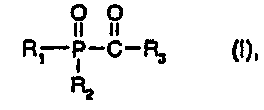 où
R₁ et R₂ représentent indépendamment l'un de l'autre un groupe alkyle en C₁-C₁₂, benzyle, phényle non substitué ou substitué une à quatre fois par des substituants halogène, alkyle en C₁-C₈ et/ou alkoxy en C₁-C₈, cyclohexyle ou un groupe COR₃ ; ou
R₁ représente un groupe -OR₄ ou un groupe 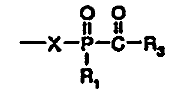 R₃ représente un groupe phényle non substitué ou substitué une à quatre fois par des substituants alkyle en C₁-C₈, alkoxy en C₁-C₈, (alkyl en C₁-C₈)thio
et/ou halogène, ou un groupe 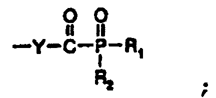 R₄ représente des groupes alkyle en C₁-C₈, phényle ou benzyle ;
Y représente des groupes phénylène, alkylène en C₁-C₁₂ ou cyclohexylène ;
X représente des groupes alkylène en C₁-C₁₈ ou un
groupe

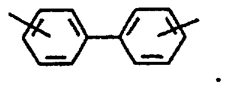

3. Combinaison complexe moléculaire selon la revendication 1, où le composé d'α-hydroxycétone est un composé de formule II 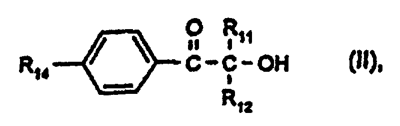 où
R₁₁ et R₁₂ représentent, indépendamment l'un de l'autre, un atome d'hydrogène, des groupes alkyle en C₁-C₆ ou phényle, ou
R₁₁ et R₁₂ forment conjointement avec l'atome de carbone auquel ils sont liés, un cycle cyclohexyle ;
R₁₄ représente un atome d'hydrogène, des groupes alkyle en C₁-C₁₈, alkoxy en C₁-C₁₂, -OCH₂CH₂-OR₁₅, un groupe CH₂=C(CH₃)- ou 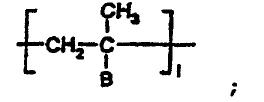 I va de 2 à 10 ;
B représente le reste de formule 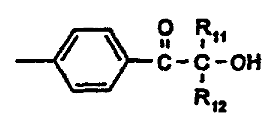 R₁₅ représente un atome d'hydrogène, des groupes

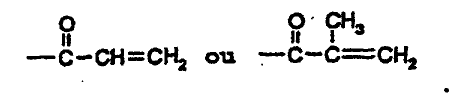

4. Combinaison complexe moléculaire selon la revendication 1, d'un composé d'oxyde de mono-, bis- ou trisacylphosphine de formule I 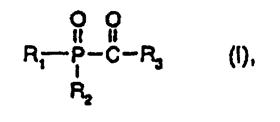 où
R₁ et R₂ représentent indépendamment l'un de l'autre, un groupe alkyle en C₁-C₁₂, un groupe phényle non substitué ou substitué une ou deux fois par des substituants alkyle en C₁-C₈, alkoxy en C₁-C₆ ou un groupe COR₃ ;
R₃ représente un reste 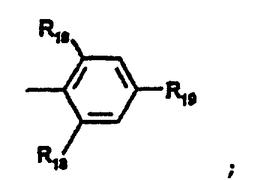 R₁₈ représente un groupe alkyle en C₁-C₄ ou alkoxy en C₁-C₄ ; et
R₁₉ représente un atome d'hydrogène ou un groupe alkyle en C₁-C₄ ;
et un composé d'α-hydroxycétone de formule II 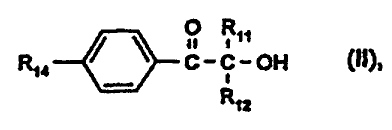 où
R₁₁ et R₁₂ représentent, indépendamment l'un de l'autre, un groupe alkyle en C₁-C₄ ou R₁₁ et R₁₂ forment conjointement avec l'atome de carbone auquel ils sont liés, un cycle cyclohexyle ; et
R₁₄ représente un atome d'hydrogène.

5. Combinaison complexe moléculaire selon la revendication 1, où le composé d'oxyde d'acylphosphine est l'oxyde de bis(2,6-diméthoxybenzoyl)-(2,4,4-triméthyl-pentyl)phosphine ou l'oxyde de 2,4,6-triméthylbenzoyl-diphénylphosphine et le composé d'α-hydroxycétone est un composé de formule II, où R₁₁ et R₁₂ représentent un groupe alkyle en C₁-C₄ ou R₁₁ et R₁₂ forment conjointement avec l'atome de carbone auquel ils sont liés un cycle cyclohexyle, et R₁₄ représente un atome d'hydrogène.

6. Combinaison complexe moléculaire selon la revendication 1, où le composé d'oxyde d'acylphosphine est l'oxyde de bis(2,6-diméthoxybenzoyl)-(2,4,4-triméthyl-pentyl)phosphine, l'oxyde de bis(2,4,6-triméthylbenzoyl)-(2,4-dihexyloxyphényl)phosphine, l'oxyde de bis(2,4,6-triméthylbenzoyl)-(4-éthoxyphényl)-phosphine ou l'oxyde de 2,4,6-triméthylbenzoyl-diphénylphosphine et le composé d'α-hydroxycétone et l'α-hydroxy-cyclohexyl-phényl-cétone ou la 2-hydroxy-2-méthyl-l-phényl-propan-1-one.

7. Combinaison complexe moléculaire selon la revendication 1, renfermant le composé d'oxyde de mono-, bis- ou trisacylphosphine et le composé d'α-hydroxycétone dans un rapport molaire de 1:1.

8. Composition photopolymérisable renfermant
(a) au moins un composé photopolymérisable à insaturation éthylénique et
(b) en tant que photoamorceur, au moins un combinaison complexe moléculaire selon la revendication 1.

9. Composition photopolymérisable selon la revendication 8, qui renferme, au côté du composant (b), d'autres additifs encore.

10. Composition photopolymérisable selon la revendication 8, présentant une teneur de 0,015 à 15, en particulier de 0,2 à 5 % en poids du composant (b) en tant que photoamorceur.

11. Composition photopolymérisable selon la revendication 8, renfermant en tant que photoamorceur au moins un combinaison complexe moléculaire selon la revendication 1, ainsi qu'un absorbeur d'UV de la catégorie des hydroxyphényl-s-triazines et/ou hydroxyphénylbenzotriazoles et/ou amines à encombrement stérique à base de 2,2,6,6-tétraméthylpipéridines.

12. Procédé pour la photopolymérisation de composés avec des doubles liaisons à insaturation éthylénique **caractérisé en ce qu'**une composition selon la revendication 8 est insolée par de la lumière dans le domaine de 200 à 600 nm.

13. Utilisation de la composition selon la revendication 8 pour la préparation de vernis, d'encres d'impression, de plaques d'impression, de matières dentaires, de matières de resist ainsi que de matière d'enregistrement d'images, en particulier pour des enregistrements holographiques.

14. Substrat revêtu sur au moins une surface par une composition selon la revendication 8.

15. Procédé pour la préparation photographique de reproductions en relief dans lequel un substrat revêtu selon la revendication 14 est insolé selon modèle et les parties non irradiées sont éliminées par un solvant.
